# EUROPEAN PATENT APPLICATION

(11) **EP 4 803 521 A1**
(43) Date of publication of application: **09.09.2026**
(21) Application number: 24885478.8
(22) Date of filing: 18.10.2024
(51) Int. Cl.: C07D 487/06, C09K 11/06, G09F 9/30, H10K 50/12, H10K 50/13, H10K 59/60, H10K 59/95, H10K 85/60, H10K 101/10

(54) **ORGANIC COMPOUND AND ORGANIC LIGHT-EMITTING ELEMENT**

(30) Priority: 30.10.2023 JP 2023185492
(71) Applicant: Canon Kabushiki Kaisha, Tokyo 146-8501 (JP)
(72) Inventor: NISHIDE, Yosuke, Tokyo 146-8501 (JP); OHRUI, Hiroki, Tokyo 146-8501 (JP); IWAWAKI, Hironobu, Tokyo 146-8501 (JP); YAMADA, Naoki, Tokyo 146-8501 (JP)
(74) Representative: TBK
(86) International application number: PCT/JP2024/037160
(87) International publication number: WO 2025/094704

(57) **Abstract**

An organic compound represented by a general formula [1]. 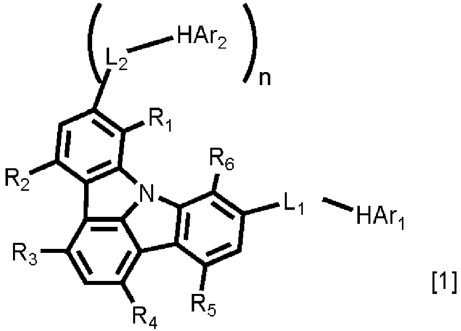 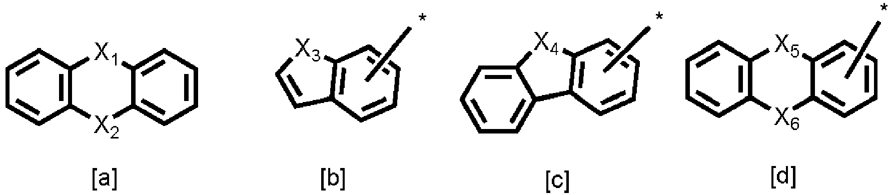 L₁ and L₂ are a direct bond or a linking group composed of at least one selected from the group consisting of a substituted or unsubstituted benzene residue, a substituted or unsubstituted residue of a compound represented by a general formula [a], and the like. In the general formula [a], X₁ and X₂ are selected from O, S, and the like. HAr₁ is selected from groups represented by general formulas [b] to [d], and HAr₂ is selected from groups represented by general formulas [e] to [g]. In the general formulas [b] to [g], X₃ to X₁₀ are selected from O, S, and the like. In the general formulas [b] to [d], * represent a bonding position with L₁. In the general formulas [e] to [g], * represent a bonding position with L₂ or H. R₁ to R₆ are each independently selected from a hydrogen atom, a deuterium atom, and the like. n is 0 or 1.

## Description

### Technical Field

The present invention relates to an organic compound and an organic light-emitting element using the organic compound.

### Background Art

Organic light-emitting elements (may also be referred to as organic electroluminescence elements or organic EL elements) are electronic elements including a pair of electrodes and an organic compound layer disposed between the electrodes. Electrons and holes are injected from the pair of electrodes to generate excitons of a light-emitting organic compound in the organic compound layer; and when the excitons return to the ground state, the organic light-emitting element emits light. Recent progress of organic light-emitting elements has been remarkable, and characteristics thereof include low driving voltage, various emission wavelengths, high-speed response, the capability of thinning and weight reduction of light-emitting devices, and the like.

Creation of compounds suitable for organic light-emitting elements has been actively conducted to date. This is because creation of compounds having improved element lifetime characteristics is important in providing high-performance organic light-emitting elements. Such a compound created is an indolocarbazole derivative 1-a, which is described in Patent Literature 1.

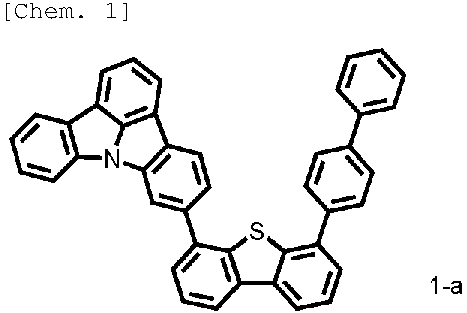

### Citation List

### Patent Literature

PTL 1: U.S. Patent Laid-Open No. 2016/0233435

### Summary of Invention

### Technical Problem

However, organic light-emitting elements containing the compound 1-a described in PTL 1 are still desired to have improved durability characteristics.

### Solution to Problem

The present invention has been made in view of the above circumstances, and an object thereof is to provide an organic compound and an organic light-emitting element having improved durability characteristics.

An organic compound according to the present invention is represented by a general formula [1] or [2] below.

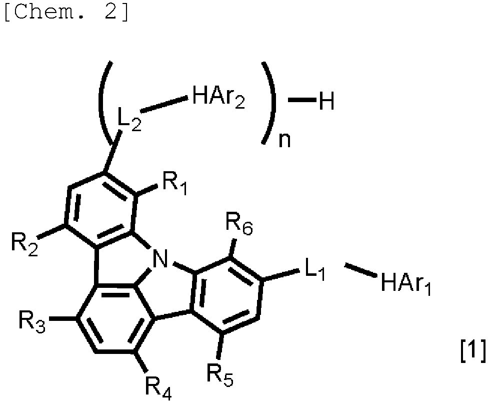

In the general formula [1], L₁ and L₂ are each independently a direct bond or a linking group composed of at least one selected from the group consisting of a substituted or unsubstituted benzene residue, a substituted or unsubstituted naphthalene residue, a substituted or unsubstituted phenanthrene residue, a substituted or unsubstituted triphenylene residue, and a substituted or unsubstituted residue of a compound represented by a general formula [a] below.

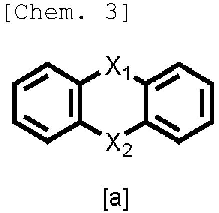

In the general formula [a], X₁ and X₂ are each independently selected from O, S, Se, and Te.

HAr₁ is selected from groups represented by general formulas [b] to [d] below, and HAr₂ is selected from groups represented by general formulas [e] to [g] below. Groups represented by general formulas [b] to [g] may further have a fused ring.

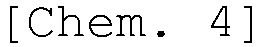

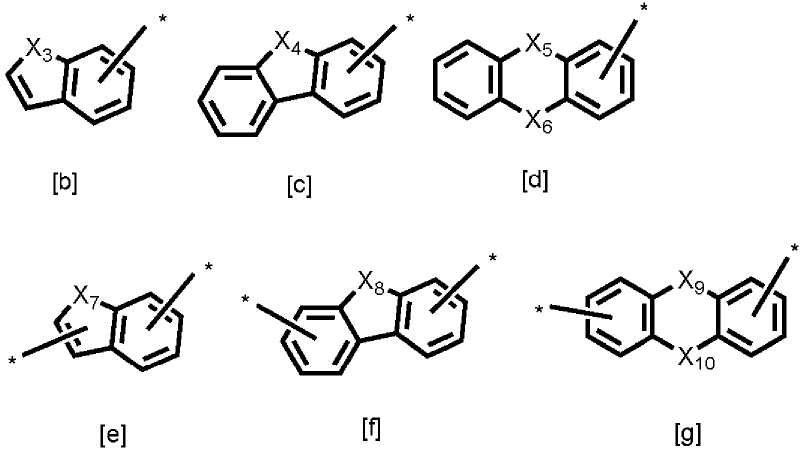

In the general formulas [b] to [g], X₃ to X₁₀ are each independently selected from O, S, Se, and Te. In the general formulas [b] to [d], * represent a bonding position with L₁. In the general formulas [e] to [g], * represent a bonding position with L₂ or H.

R₁ to R₆ are each independently selected from a hydrogen atom, a deuterium atom, a halogen atom, a substituted or unsubstituted alkyl group, a substituted or unsubstituted alkoxy group, and a substituted or unsubstituted silyl group.

n is 0 or 1.

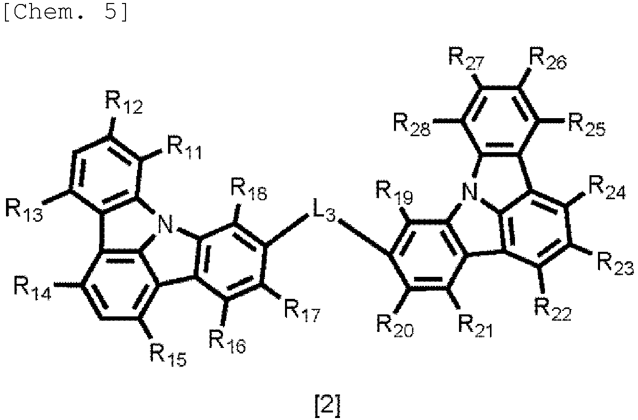

In the general formula [2], L₃ is a linking group composed of at least one selected from the group consisting of a substituted or unsubstituted benzene residue, a substituted or unsubstituted naphthalene residue, a substituted or unsubstituted phenanthrene residue, a substituted or unsubstituted triphenylene residue, and substituted or unsubstituted groups represented by general formulas [h] to [j] below.

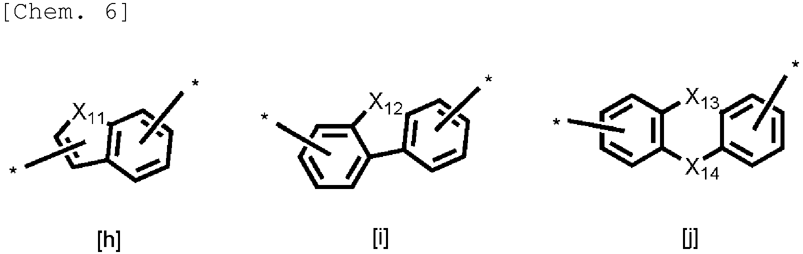

In the general formulas [h] to [j], X₁₁ to X₁₄ are each independently selected from O, S, Se, and Te, and * represent a bonding position.

R₁₁ to R₂₈ are each independently selected from a hydrogen atom, a deuterium atom, a halogen atom, a substituted or unsubstituted alkyl group, a substituted or unsubstituted alkoxy group, and a substituted or unsubstituted silyl group.

### Advantageous Effects of Invention

The organic compound according to the present invention can provide an organic light-emitting element having improved durability characteristics.

### Brief Description of Drawings

[Fig. 1A] Fig. 1A is a schematic sectional view illustrating an example of a pixel of a display device according to an embodiment of the present invention.
[Fig. 1B] Fig. 1B is a schematic sectional view of an example of a display device using an organic light-emitting element according to an embodiment of the present invention.
[Fig. 2] Fig. 2 is a schematic view illustrating an example of a display device according to an embodiment of the present invention.
[Fig. 3A] Fig. 3A is a schematic view illustrating an example of an image pickup device according to an embodiment of the present invention.
[Fig. 3B] Fig. 3B is a schematic view illustrating an example of an electronic apparatus according to an embodiment of the present invention.
[Fig. 4A] Fig. 4A is a schematic view illustrating an example of a display device according to an embodiment of the present invention.
[Fig. 4B] Fig. 4B is a schematic view illustrating an example of a foldable display device.
[Fig. 5A] Fig. 5A is a schematic view illustrating an example of a lighting apparatus according to an embodiment of the present invention.
[Fig. 5B] Fig. 5B is a schematic view illustrating an example of a mobile object including a vehicle lighting device according to an embodiment of the present invention.
[Fig. 6A] Fig. 6A is a schematic view illustrating an example of a wearable device according to an embodiment of the present invention.
[Fig. 6B] Fig. 6B is a schematic view illustrating another example of a wearable device according to an embodiment of the present invention.
[Fig. 7A] Fig. 7A is a schematic view illustrating an example of an image forming apparatus according to an embodiment of the present invention.
[Fig. 7B] Fig. 7B is a schematic view illustrating an example of an exposure light source of an image forming apparatus according to an embodiment of the present invention.
[Fig. 7C] Fig. 7C is a schematic view illustrating an example of an exposure light source of an image forming apparatus according to an embodiment of the present invention.

### Description of Embodiments

### <Organic compound>

First, an organic compound according to the present embodiment will be described. The organic compound of the present embodiment is represented by the following general formula [1] or [2].

### <Organic compound represented by general formula [1]>

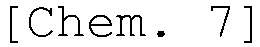

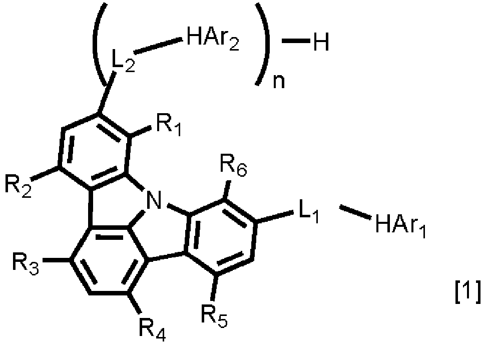

### [L₁ and L₂]

In the general formula [1], L₁ and L₂ are each independently a direct bond or a linking group composed of at least one selected from the group consisting of a substituted or unsubstituted benzene residue, a substituted or unsubstituted naphthalene residue, a substituted or unsubstituted phenanthrene residue, a substituted or unsubstituted triphenylene residue, and a substituted or unsubstituted residue of a compound represented by a general formula [a] below. Here, the linking group is a group composed of one group or a plurality of groups selected from the benzene residue and the like and bonded together; for example, in Exemplified Compound A7 described later, the linking group L₁ is composed of five benzene ring residues.

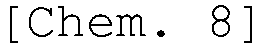

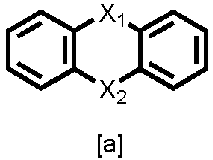

In the general formula [a], X₁ and X₂ are each independently selected from O, S, Se, and Te. X₁ and X₂ are each preferably independently selected from O and S.

L₁ and L₂ are each independently preferably a direct bond or a linking group composed of at least one selected from the group consisting of a substituted or unsubstituted benzene residue having a valence of three or less, preferably a valence of two or less, a substituted or unsubstituted naphthalene residue having a valence of three or less, preferably a valence of two or less, a substituted or unsubstituted phenanthrene residue having a valence of three or less, preferably a valence of two or less, a substituted or unsubstituted triphenylene residue having a valence of three or less, preferably a valence of two or less, and a substituted or unsubstituted residue of the compound represented by the general formula [a], having a valence of three or less, preferably a valence of two or less.

L₁ and L₂ are each independently preferably a linking group composed of at least one selected from the group consisting of a substituted or unsubstituted benzene residue, a substituted or unsubstituted naphthalene residue, a substituted or unsubstituted phenanthrene residue, and a substituted or unsubstituted triphenylene residue.

The benzene residue is preferably a residue bonded at a meta position, and L₁ and L₂ are preferably a linking group composed of one or more m-phenylene groups.

Non-limiting examples of substituents that residues constituting the linking group may include include a deuterium atom and heterocyclic groups such as a group represented by a general formula [b] below and a group represented by a general formula [c] below.

### [HAr₁ and HAr₂]

In the general formula [1], HAr₁ is selected from groups represented by the following general formulas [b] to [d], and HAr₂ is selected from groups represented by the following general formulas [e] to [g]. HAr₁ is preferably a group represented by the following general formula [c] or [d], and HAr₂ is preferably a group represented by the following general formula [f] or [g].

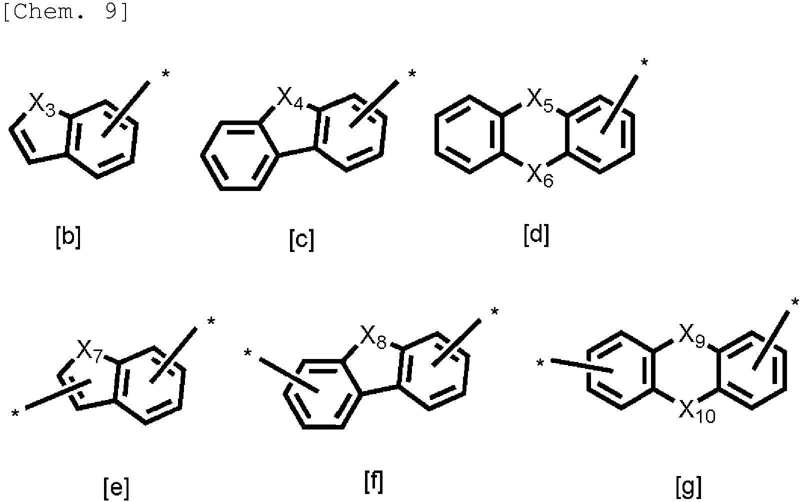

In the general formulas [b] to [g], X₃ to X₁₀ are each independently selected from O, S, Se, and Te. X₃ to X₁₀ are each preferably independently selected from O and S.

In the general formulas [b] to [d], * represent a bonding position with L₁. In the general formulas [e] to [g], * represent a bonding position with L₂ or H.

The groups represented by the general formulas [b] to [d] are a monovalent group, and the bonding position * may be on any carbon atom. For example, in the group represented by the general formula [b], the bonding position

* may be on either a carbon atom constituting the five-membered ring or a carbon atom constituting the six-membered ring. The groups represented by the general formulas [e] to [g] are a divalent group, and the two bonding positions * may be on any carbon atom. For example, in the group represented by the general formula [e], the two bonding positions * may be on one carbon atom constituting the five-membered ring and one carbon atom constituting the six-membered ring, or may be on two carbon atoms within either carbon atoms constituting the five-membered ring or carbon atoms constituting the six-membered ring.

The groups represented by the general formulas [b] to [g] may further include a fused ring. Non-limiting examples of the fused ring that may be further included include aromatic rings such as a benzene ring, a naphthalene ring, and a phenanthrene ring, and aromatic heterocycles such as a thiophene ring, a benzothiophene ring, a dibenzothiophene ring, a furan ring, a benzofuran ring, and a dibenzofuran ring.

### [R₁ to R₆]

In the general formula [1], R₁ to R₆ are each independently selected from a hydrogen atom, a deuterium atom, a halogen atom, a substituted or unsubstituted alkyl group, a substituted or unsubstituted alkoxy group, and a substituted or unsubstituted silyl group.

Non-limiting examples of the halogen atom include fluorine, chlorine, bromine, iodine, astatine, and tennessine.

The alkyl group may be an alkyl group having 1 or more and 20 or less carbon atoms. Non-limiting examples include a methyl group, an ethyl group, a n-propyl group, an isopropyl group, a n-butyl group, a tert-butyl group, a sec-butyl group, an octyl group, a cyclohexyl group, a tert-pentyl group, a 3-methylpentan-3-yl group, a 1-adamantyl group, and a 2-adamantyl group.

The alkoxy group may be an alkoxy group having 1 or more and 10 or less carbon atoms. Non-limiting examples include a methoxy group, an ethoxy group, a propoxy group, an isoprooxy group, a tert-butoxy group, a 2-ethyl-octyloxy group, and a benzyloxy group.

Non-limiting examples of the silyl group include a trimethylsilyl group and a triphenylsilyl group.

Non-limiting examples of substituents that the alkyl group, the alkoxy group, and the silyl group may further include include deuterium, alkyl groups such as a methyl group, an ethyl group, a n-propyl group, an isopropyl group, a n-butyl group, and a tert-butyl group, aralkyl groups such as a benzyl group, aryl groups such as a phenyl group and a biphenyl group, heterocyclic groups such as a pyridyl group and a pyrrolyl group, amino groups such as a dimethylamino group, a diethylamino group, a dibenzylamino group, a diphenylamino group, and a ditolylamino group, alkoxy groups such as a methoxy group, an ethoxy group, and a propoxy group, aryloxy groups such as a phenoxy group, halogen atoms such as fluorine, chlorine, bromine, and iodine, and a cyano group.

### [n]

n is 0 or 1. n is preferably 0.

### <Organic compound represented by general formula [2]>

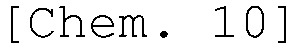

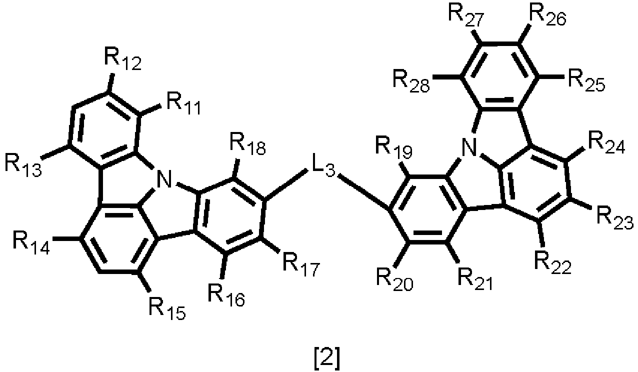

### [L₃]

In the general formula [2], L₃ is a linking group composed of at least one selected from the group consisting of a substituted or unsubstituted benzene residue, a substituted or unsubstituted naphthalene residue, a substituted or unsubstituted phenanthrene residue, a substituted or unsubstituted triphenylene residue, and substituted or unsubstituted groups represented by the following general formulas [h] to [j]. Here, the linking group is a group composed of one group or a plurality of groups selected from the benzene residue and the like and bonded together; for example, in Exemplified Compound D3 described later, the linking group L₃ is composed of two benzene ring residues.

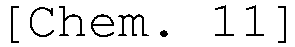

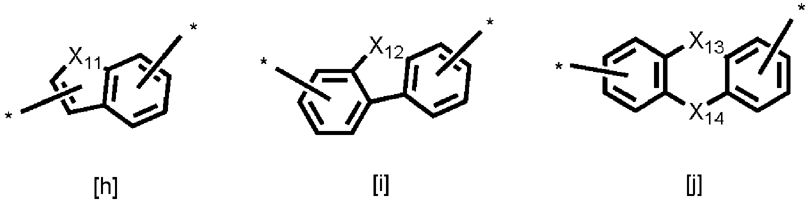

In the general formulas [h] to [j], X₁₁ to X₁₄ are each independently selected from O, S, Se, and Te. X₁₁ to X₁₄ are each preferably independently selected from O and S.

In the general formulas [h] to [j], * represent a bonding position. The groups represented by the general formulas [h] to [j] are a divalent group, and the two bonding positions * may be on any carbon atom. For example, in the group represented by the general formula [h], the two bonding positions * may be on one carbon atom constituting the five-membered ring and one carbon atom constituting the six-membered ring, or may be on two carbon atoms within either carbon atoms constituting the five-membered ring or carbon atoms constituting the six-membered ring.

L₃ is preferably a linking group composed of at least one selected from the group consisting of a substituted or unsubstituted benzene residue having a valence of three or less, preferably a valence of two or less, a substituted or unsubstituted naphthalene residue having a valence of three or less, preferably a valence of two or less, a substituted or unsubstituted phenanthrene residue having a valence of three or less, preferably a valence of two or less, a substituted or unsubstituted triphenylene residue having a valence of three or less, preferably a valence of two or less, and substituted or unsubstituted groups represented by the general formulas [h] to [j].

Non-limiting examples of substituents that a group constituting the linking group may include include a deuterium atom.

### [R₁₁ to R₂₈]

In the general formula [2], R₁₁ to R₂₈ are each independently selected from a hydrogen atom, a deuterium atom, a halogen atom, a substituted or unsubstituted alkyl group, a substituted or unsubstituted alkoxy group, and a substituted or unsubstituted silyl group.

Non-limiting examples of the alkyl group, the alkoxy group, and the silyl group include the groups having been described for R₁ to R₆. Non-limiting examples of substituents that the alkyl group, the alkoxy group, and the silyl group may further include include the groups having been described for R₁ to R₆.

### <Features>

The organic compound of the present embodiment has the following features.
(1-1) By including at least one indolocarbazole unit and including heteroaryl groups at two terminal units of the molecule, compatibility is improved, accumulation of charges and energy is addressed, and improved durability characteristics are provided. Alternatively, by including at least one indolocarbazole unit and including heteroaryl groups at two terminal units of the molecule, the molecule has a large permanent dipole moment and durability is improved.
(1-2) By including hole-transporting heteroaryl groups at two terminal units of the molecule, hole-transporting capability is improved and a lower driving voltage is achieved.

Hereinafter, these features will be described with reference to, as a comparative control, a comparative compound having a structure similar to that of the organic compound of the present embodiment, and properties of the basic skeleton of the organic compound according to the present embodiment will be described. Specifically, Comparative Compound 1-a and Exemplified Compounds of the present embodiment will be presented.

(1-1) By including at least one indolocarbazole unit and including heteroaryl groups at two terminal units of the molecule, compatibility is improved, accumulation of charges and energy is addressed, and improved durability characteristics are provided. Alternatively, by including at least one indolocarbazole unit and including heteroaryl groups at two terminal units of the molecule, the molecule has a large permanent dipole moment and durability is improved.

The organic compound of the present embodiment includes an indolocarbazole unit. The indolocarbazole unit has high hole-transporting performance and has no rotational axis and thus has high structural stability. The compound represented by the general formula [1] has one indolocarbazole unit. When n = 0, terminal units of the molecule are the indolocarbazole unit and HAr₁, and both are heteroaryl groups. Alternatively, when n = 1, terminal units of the molecule are HAr₁ and HAr₂, and both are heteroaryl groups. On the other hand, the compound represented by the general formula [2] has two indolocarbazole units. The terminal units of the molecule are the indolocarbazole units, which are heteroaryl groups.

In inventing the organic compound represented by the general formula [1] or [2], the present inventors focused on terminal units of the molecule and the permanent dipole moment.

A compound in an organic layer, particularly in a light-emitting layer, of an organic light-emitting element undergoes repeated transitions between the ground state and the excited state in a light-emission process of the organic light-emitting element. In particular, in an organic phosphorescent light-emitting element, it is important to control the triplet excited state (T₁), which accounts for 75% of the excited states. For example, energy transfer needs to be efficiently promoted from T₁ of a host molecule to a guest molecule and to cause the guest molecule to emit light efficiently. When energy transfer efficiency is low, a probability that generated excitation energy is used for a reaction with an adjacent molecule is increased, thereby causing deterioration of durability characteristics due to generation of a quencher molecule. The energy transfer process from T₁ of the host molecule to the guest molecule is known to occur by Dexter energy transfer. In order to improve efficiency of Dexter energy transfer, it is important to shorten the distance between the host molecule and the guest molecule as much as possible.

The present inventors performed thorough studies and, as a result, have found that, in order to shorten the intermolecular distance between the host molecule and the guest molecule, it is effective to include at least one indolocarbazole unit and to include heteroaryl groups at two terminal units of the molecule. This is inferentially because heteroatom units that suitably interact with a metal atom (for example, an iridium atom) of the guest molecule are disposed at both terminals of the molecule, so that intermolecular interaction with the guest molecule is increased and the intermolecular distance is shortened. In order to shorten the intermolecular distance as much as possible, the terminal heteroaryl units are preferably unsubstituted. Specifically, in the compound represented by the general formula [1] in which n = 0, R₁ to R₆ are preferably hydrogen atoms. The compound represented by the general formula [1] in which n = 1 is preferred because HAr₁ and HAr₂ are unsubstituted. In the compound represented by the general formula [2], R₁₁ to R₂₈ are preferably hydrogen atoms.

From another viewpoint, heterocycles, which have heteroatoms in the skeletons, have large polarization. That is, the organic compound of the present embodiment has a large permanent dipole moment. Further, a host molecule having a large permanent dipole moment has high compatibility with guest molecules having high polarity such as Ir complexes and Pt complexes and hence is preferred. Table 1 describes, for Exemplified Compounds B27 and E12 of the present embodiment and Comparative Compound 1-a, values obtained by calculating permanent dipole moments by molecular orbital calculation and the number of terminal hetero units. Further, Table 1 describes durability characteristics (luminance decrease ratio) when used as the hosts of organic light-emitting elements in Examples (Examples 35 and 53 and Comparative Example 1).

**[Table 1]**

| | Molecular structure | Dipole moment | Hetero structure | Element durability |
|---|---|---|---|---|
| Present invention 1 | 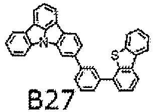 | 0.9 (Calc.) | 2 terminals | 1.3 |
| Present invention 2 | 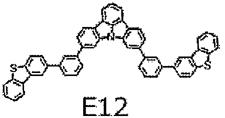 | 1.4 (Calc.) | 2 terminals | 1.2 |
| Comparative 1 | 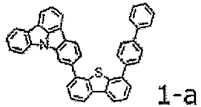 | 0.6 (Calc.) | 1 terminal | 1.0 |

As described in Table 1, Exemplified Compounds B27 and E12, compared with Comparative Compound 1-a, have increased numbers of terminal hetero units and increased dipole moments to thereby have improved durability.

As has been described, by including at least one indolocarbazole unit and including heteroaryl groups at two terminal units of the molecule, compatibility is improved, accumulation of charges and energy is addressed, and durability characteristics are improved. Alternatively, by including at least one indolocarbazole unit and including heteroaryl groups at two terminal units of the molecule, the molecule has a large permanent dipole moment and durability is improved.

(1-2) By including hole-transporting heteroaryl groups at two terminal units of the molecule, hole-transporting capability is improved and a lower driving voltage is achieved.

The organic compound of the present embodiment can be used for a hole transport layer, an electron blocking layer, a light-emitting layer, other functional layers, and the like of an organic light-emitting element, and, in particular, can be suitably used as the host of the light-emitting layer. Further, the organic compound of the present embodiment has a high T₁ (lowest triplet excitation energy) and can be suitably used as the host of a light-emitting layer of a system using a triplet excited state for light emission, such as phosphorescence and delayed fluorescence. Further, the organic compound of the present embodiment has a shallow HOMO (highest occupied molecular orbital) (close to the vacuum level) and has high hole-transporting capability, and thus can be used in mixture with an electron-transporting host.

The organic compound of the present embodiment includes an indolocarbazole unit and has high hole-transporting performance. Here, the compound represented by the general formula [2] has indolocarbazole units at two terminal units of the molecule. However, in the compound represented by the general formula [1], at least one of terminal units of the molecule is not an indolocarbazole unit but is HAr₁ or HAr₂. Thus, the heteroaryl groups represented by HAr₁ and HAr₂ preferably also have hole-transporting performance. Examples thereof include furan, benzofuran, dibenzofuran, thiophene, benzothiophene, dibenzothiophene, thianthrene, and units in which the foregoing are fused together. Note that azine-based units such as pyridine, pyrazine, pyrimidine, and triazine, azole-based units such as imidazole, oxazole, and thiazole, ketone-containing units, and the like are electron-transporting units and have low hole-transporting performance, and thus are not preferred. Accordingly, in the organic compound of the present embodiment, HAr₁ is selected from the groups represented by the general formulas [b] to [d], and HAr₂ is selected from the groups represented by the general formulas [e] to [g].

Further, in order to improve mobility, a terminal heteroaryl unit is preferably unsubstituted. Specifically, as described above, in the compound represented by the general formula [1] in which n = 0, R₁ to R₆ are preferably hydrogen atoms. The compound represented by the general formula [1] in which n = 1 is preferred because HAr₁ and HAr₂ are unsubstituted. In the compound represented by the general formula [2], R₁₁ to R₂₈ are preferably hydrogen atoms.

As has been described, by including both an indolocarbazole unit and a hole-transporting heteroaryl group, hole-transporting capability is improved and mobility is improved, and thus the voltage of an organic light-emitting element can be reduced. Further, the larger the number of hole-transporting units disposed at terminals of the molecule, the higher the effect. For example, the driving voltages at 100 mA/cm² of organic light-emitting elements of Examples (Example 35 and Comparative Example 1) using Exemplified Compound B27 and Comparative Compound 1-a were measured, and values when the voltage of Comparative Example 1 is taken as 1.0 are described in Table 2. As described in Table 2, comparison of both indicates that Exemplified Compound B27 was found to exhibit a lower value of 0.95.

**[Table 2]**

| | Molecular structure | Dipole moment | Hetero structure | Driving voltage |
|---|---|---|---|---|
| Present invention 1 | 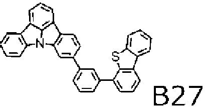 | 0.9 (Calc.) | 2 terminals | 0.95 |
| Comparative 1 | 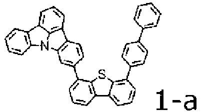 | 0.6 (Calc.) | 1 terminal | 1.0 |

Note that the permanent dipole moments in Table 1 and Table 2 were calculated using molecular orbital calculation. The calculation method of the molecular orbital calculation employed was density functional theory (Density Functional Theory, DFT), which is currently widely used. The functional was B3LYP and the basis set was 6-31G*. Note that the molecular orbital calculation was carried out using Gaussian 09, which is currently widely used (Gaussian 09, Revision C.01, M. J. Frisch, G. W. Trucks, H. B. Schlegel, G. E. Scuseria, M. A. Robb, J. R. Cheeseman, G. Scalmani, V. Barone, B. Mennucci, G. A. Petersson, H. Nakatsuji, M. Caricato, X. Li, H. P. Hratchian, A. F. Izmaylov, J. Bloino, G. Zheng, J. L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J. A. Montgomery, Jr., J. E. Peralta, F. Ogliaro, M. Bearpark, J. J. Heyd, E. Brothers, K. N. Kudin, V. N. Staroverov, T. Keith, R. Kobayashi, J. Normand, K. Raghavachari, A. Rendell, J. C. Burant, S. S. Iyengar, J. Tomasi, M. Cossi, N. Rega, J. M. Millam, M. Klene, J. E. Knox, J. B. Cross, V. Bakken, C. Adamo, J. Jaramillo, R. Gomperts, R. E. Stratmann, O. Yazyev, A. J. Austin, R. Cammi, C. Pomelli, J. W. Ochterski, R. L. Martin, K. Morokuma, V. G. Zakrzewski, G. A. Voth, P. Salvador, J. J. Dannenberg, S. Dapprich, A. D. Daniels, O. Farkas, J. B. Foresman, J. V. Ortiz, J. Cioslowski, and D. J. Fox, Gaussian, Inc., Wallingford CT, 2010.). Hereinafter, molecular orbital calculations performed herein use the same method.

Further, the organic compound of the present embodiment preferably has the following features.
(1-3) All freely rotatable single bonds are bonds between sp² carbons, so that the bond energy is high and element durability is improved.
(1-4) In the general formula [1], L₁ and L₂ are linking groups composed only of hydrocarbons, so that the effect of the terminal heteroaryl groups can be enhanced. Further, more preferably, L₁ and L₂ are linking groups composed of one or more m-phenylene groups, so that T₁ is increased and element efficiency and element durability are improved.
(1-5) In the general formula [1], n is 0, so that T₁ is increased and element efficiency and element durability are improved.

Hereinafter, these features will be described.

(1-3) All freely rotatable single bonds are bonds between sp² carbons, so that the bond energy is high and element durability is improved.

A compound in an organic layer, particularly in a light-emitting layer, of an organic light-emitting element undergoes repeated transitions between the ground state and the excited state in a light-emission process of the organic light-emitting element. In this process, vigorous motions such as expansion and contraction and rotation of the molecule occur. At that time, when there is a site where a bond is easily dissociated, the bond may be cleaved and a part of the compound may be released. When a part of the compound is released, the structure is changed; and thus the compound that tends to undergo the release has low durability. Further, when such a compound is used for an organic light-emitting element, the released part acts as a quencher and decreases element durability. Therefore, a molecule having a structure in which bonds are not easily dissociated and the release does not easily occur has higher durability.

In the organic compound of the present embodiment, all freely rotatable single bonds are bonds between sp² carbons, so that release due to cleavage of a bond does not easily occur and durability is high. Therefore, when the compound according to the present embodiment is used in an organic layer of an organic light-emitting element, release due to cleavage of a bond during driving of the element does not easily occur, so that deterioration of the element is suppressed even when driven for a long time, to provide an organic light-emitting element having high durability.

(1-4) In the general formula [1], L₁ and L₂ are linking groups composed only of hydrocarbons, so that the effect of the terminal heteroaryl groups can be enhanced. Further, more preferably, L₁ and L₂ are linking groups composed of one or more m-phenylene groups, so that T₁ is increased and element efficiency and element durability are improved.

As described in (1-1), the organic compound of the present embodiment has, due to the effect of heteroatoms at terminals of the molecule, improved compatibility with a guest molecule. Thus, when units other than terminals of the molecule are composed of units having low polarity, the effect of polarity at terminals of the molecule tends to be provided. Accordingly, L₁ and L₂ are preferably linking groups composed only of hydrocarbons, which have low polarity. Specifically, L₁ and L₂ are each independently preferably a linking group composed of at least one selected from the group consisting of a substituted or unsubstituted benzene residue, a substituted or unsubstituted naphthalene residue, a substituted or unsubstituted phenanthrene residue, and a substituted or unsubstituted triphenylene residue.

Further, L₁ and L₂ are preferably a linking group composed of one or more m-phenylene groups because T₁ is increased. For example, as described in Table 3, Exemplified Compound B31 in which L₁ is composed of two m-phenylene groups has a higher T₁ than Comparative Compound 1-a in which L₁ is composed of a heteroarylene group. With a higher T₁, energy transfer efficiency to a guest molecule is increased, and thus element efficiency is improved; and an exciton lifetime is shortened, and thus element durability is also improved. Note that T₁ in Table 3 indicates the wavelength at rise of a peak when a single film is formed and measured for an emission spectrum at 77 K.

**[Table 3]**

| | Molecular structure | T1 at rise (film)/nm |
|---|---|---|
| Present invention 3 | 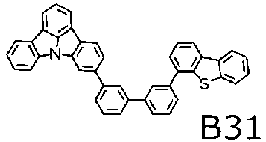 | 494 |
| Comparative 1 | 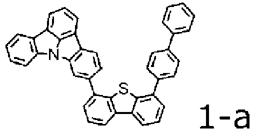 | 501 |

(1-5) In the general formula [1], n is 0, so that T₁ is increased and element efficiency and element durability are improved.

As described above, the organic compound of the present embodiment has a high T₁. Further, in the case of the organic compound of the present embodiment that is represented by the general formula [1] in which n = 0, T₁ is further increased. Results of molecular orbital calculation are described in Table 4. As described in Table 4, for example, Exemplified Compound B27 represented by the general formula [1] in which n = 0 has a higher T₁ than Exemplified Compound E9 represented by the general formula [1] in which n = 1, and has improved element efficiency and improved element durability, which is preferred. Note that T₁ in Table 4 indicates wavelengths at rise of peaks similar to T₁ in Table 3.

**[Table 4]**

| | Molecular structure | T1 at rise (film)/nm |
|---|---|---|
| Present invention 1 | 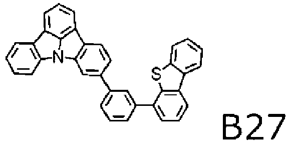 | 494 |
| Present invention 4 | 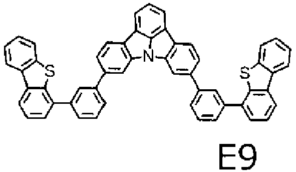 | 498 |

HOMO and LUMO described herein can be calculated using an ionization potential and a bandgap.

The HOMO can be estimated by measuring an ionization potential. The ionization potential can be measured with a measuring apparatus such as an AC-3 after dissolving a compound to be measured in a solvent such as toluene, or after depositing the compound to be measured on a substrate of glass or the like.

The LUMO can be calculated using values of the bandgap and the ionization potential. The value of the ionization potential can be subtracted from the bandgap to thereby estimate the LUMO. The bandgap can be measured by a measurement in which a compound to be measured is dissolved in a solvent such as toluene and irradiated with excitation light. The absorption edge of an excitation-light absorption spectrum can be measured to thereby measure the bandgap. Alternatively, the bandgap can be measured by depositing a compound to be measured on a substrate of glass or the like and irradiating the deposited film with excitation light. The absorption edge of an absorption spectrum in which the deposited film absorbs the excitation light can be measured to thereby measure the bandgap.

The LUMO can also be estimated from a reduction potential. For example, a one-electron reduction potential is estimated using CV (cyclic voltammetry) measurement. CV measurement can be carried out, for example, in a DMF solution of 0.1 M tetrabutylammonium perchlorate, using Ag/Ag⁺ as a reference electrode, Pt as a counter electrode, and glassy carbon as a working electrode. The difference of the determined reduction potential of the compound from the reduction potential of ferrocene can be added to -4.8 eV to estimate the LUMO.

### <Specific examples>

Specific examples of the organic compound according to the present embodiment will be described below. However, the present embodiment is not limited thereto.

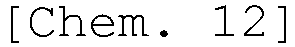

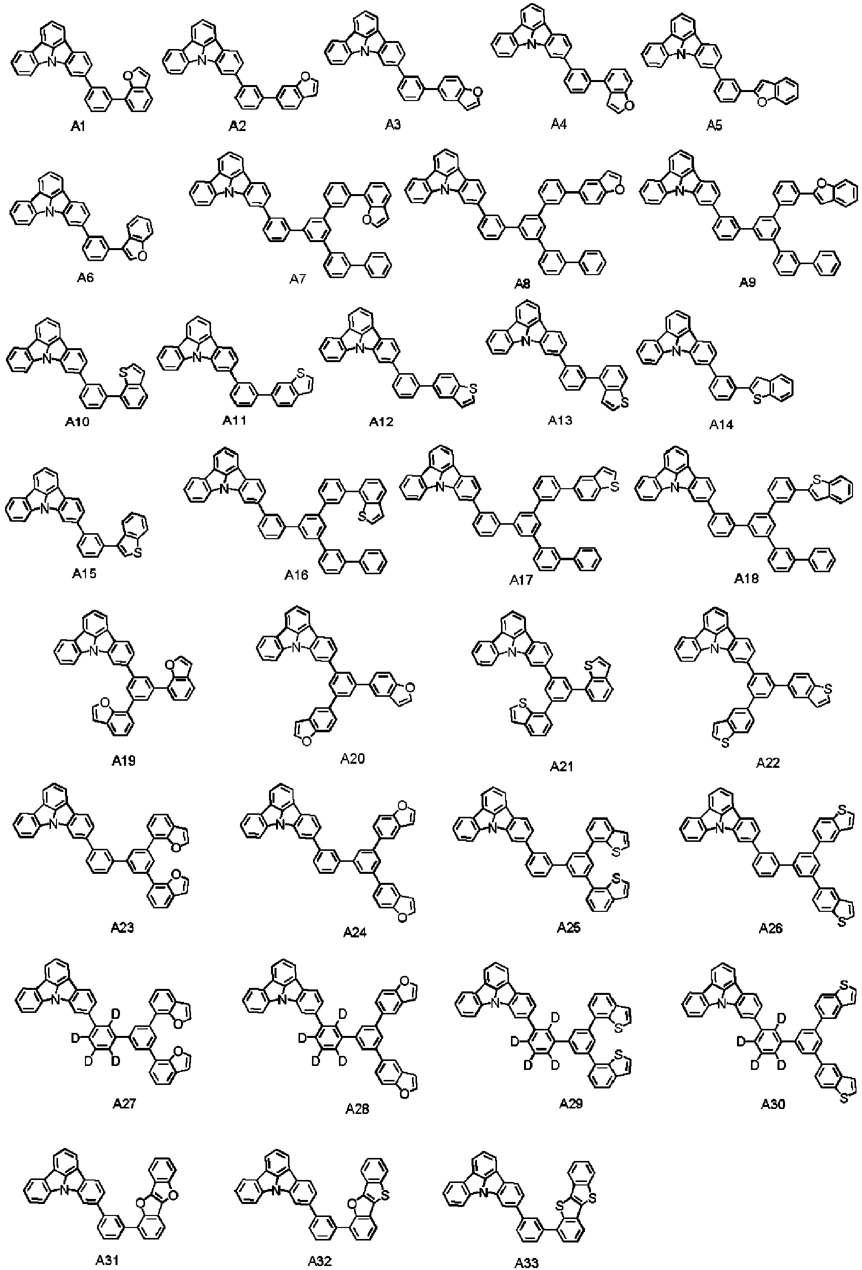

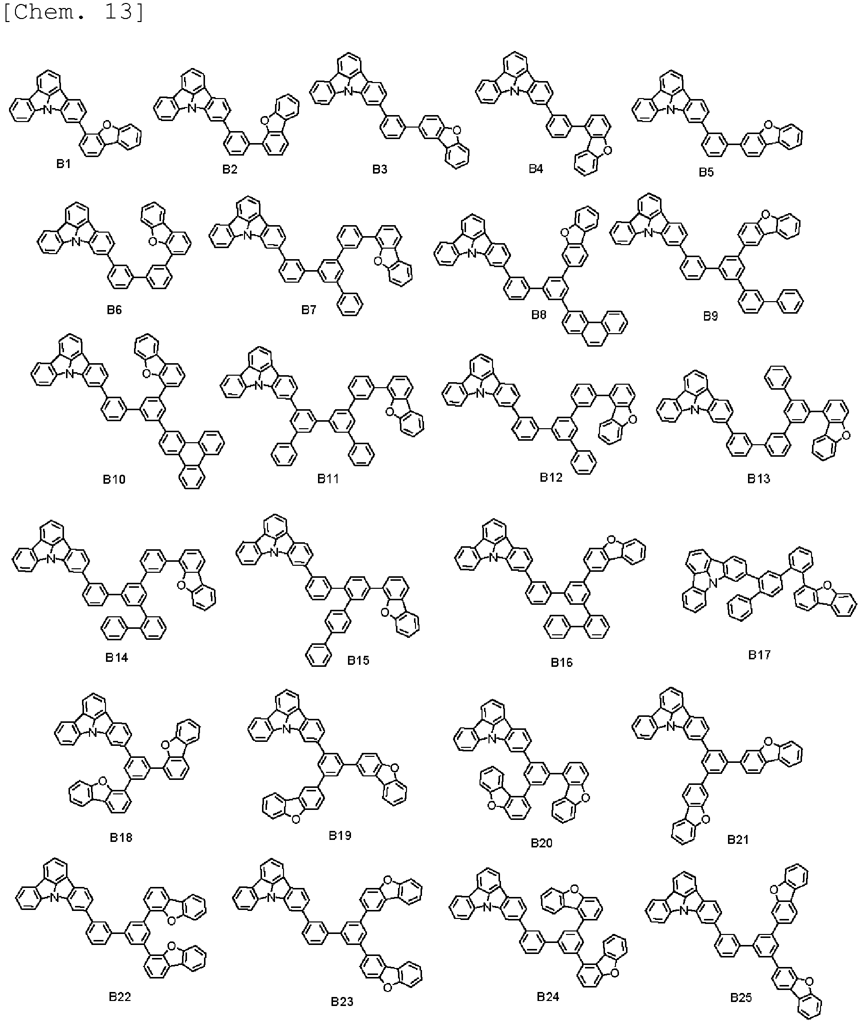

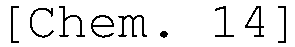

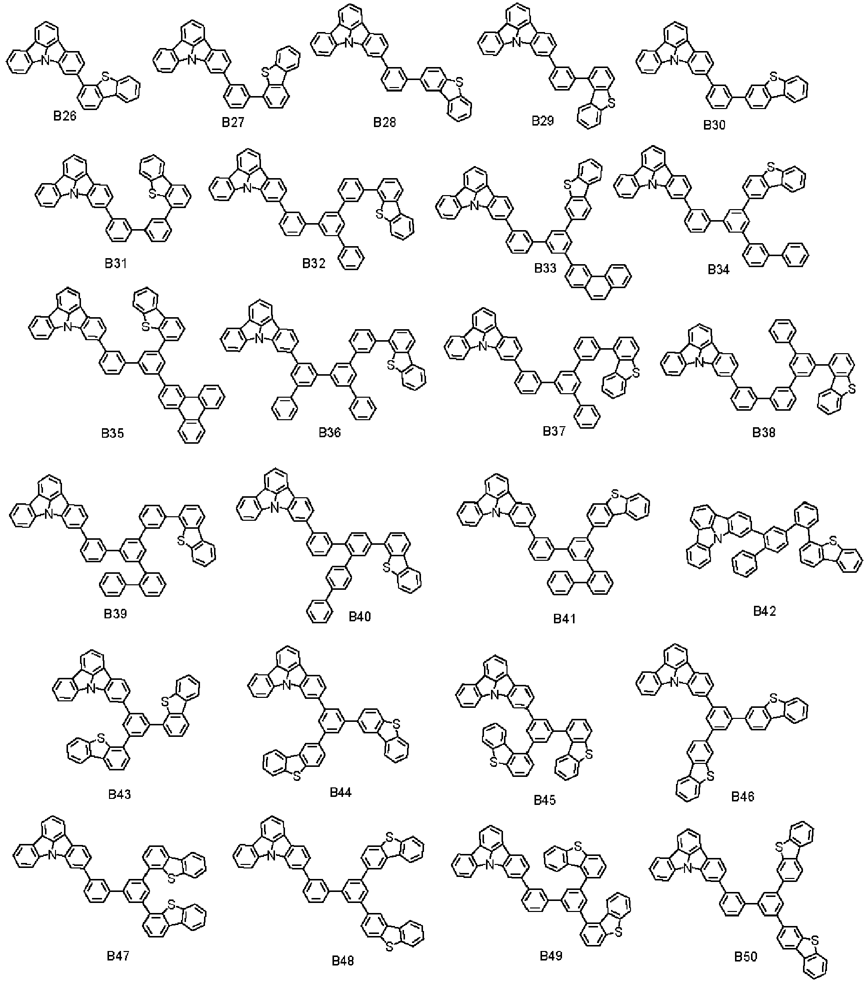

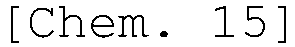

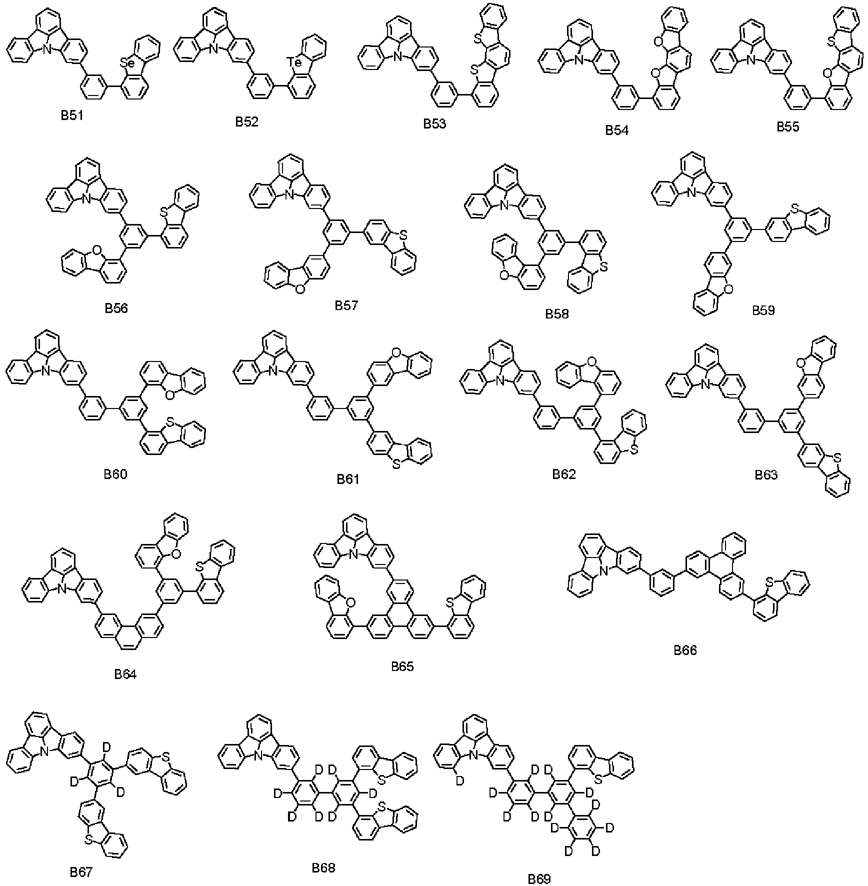

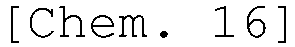

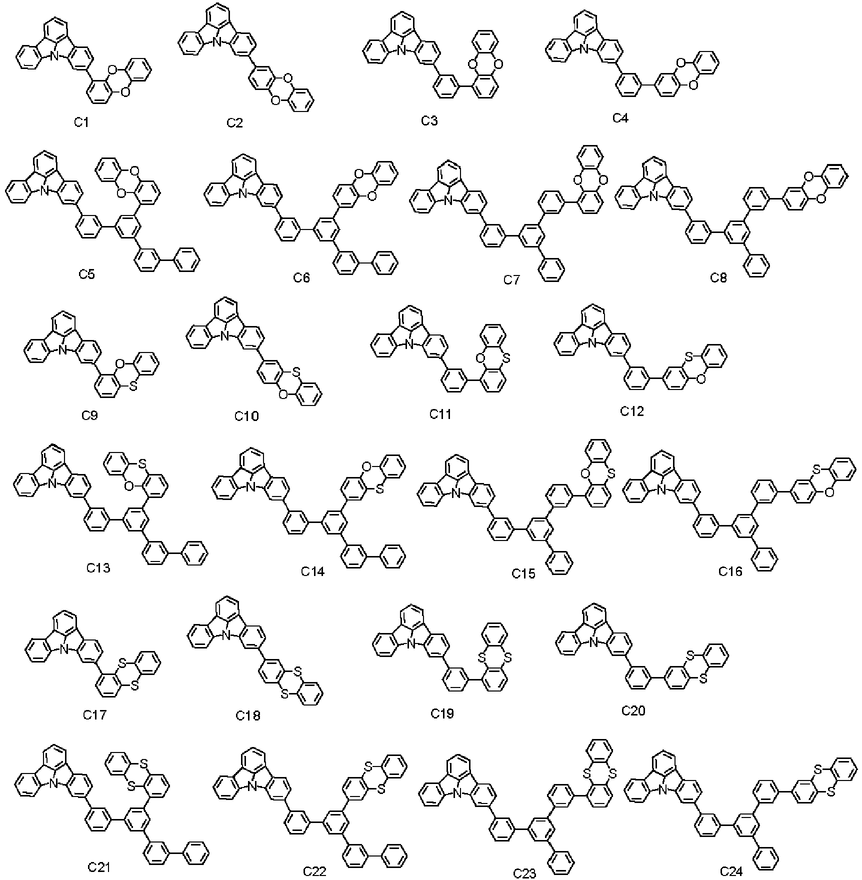

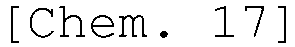

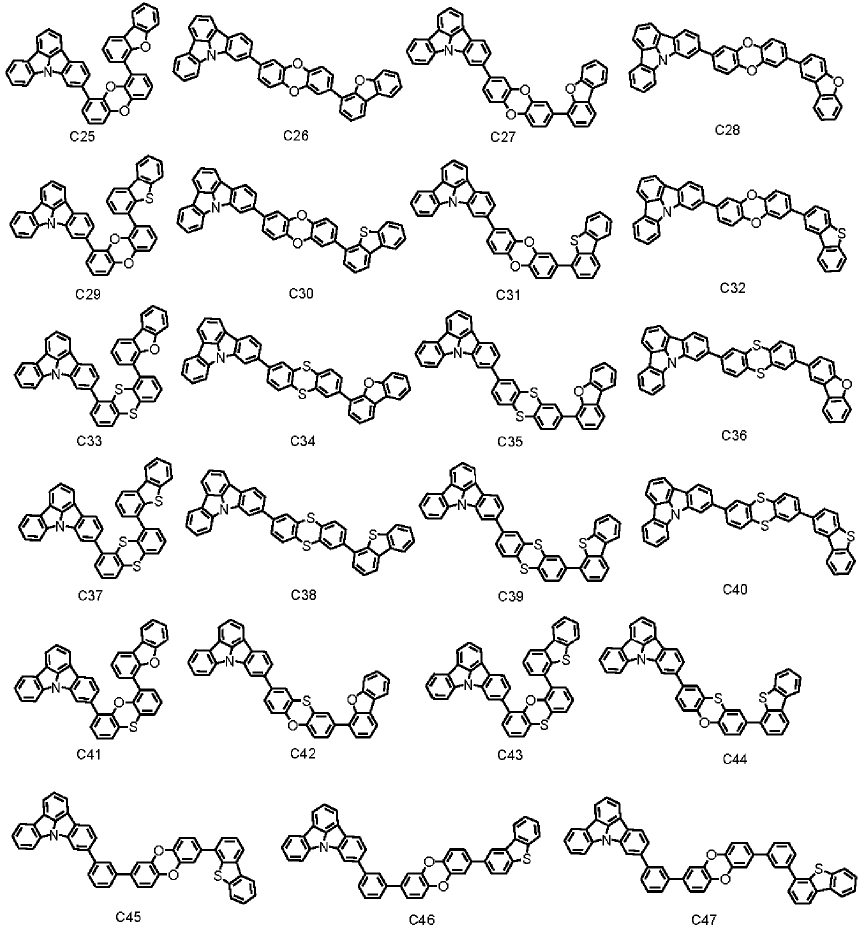

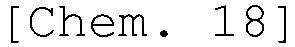

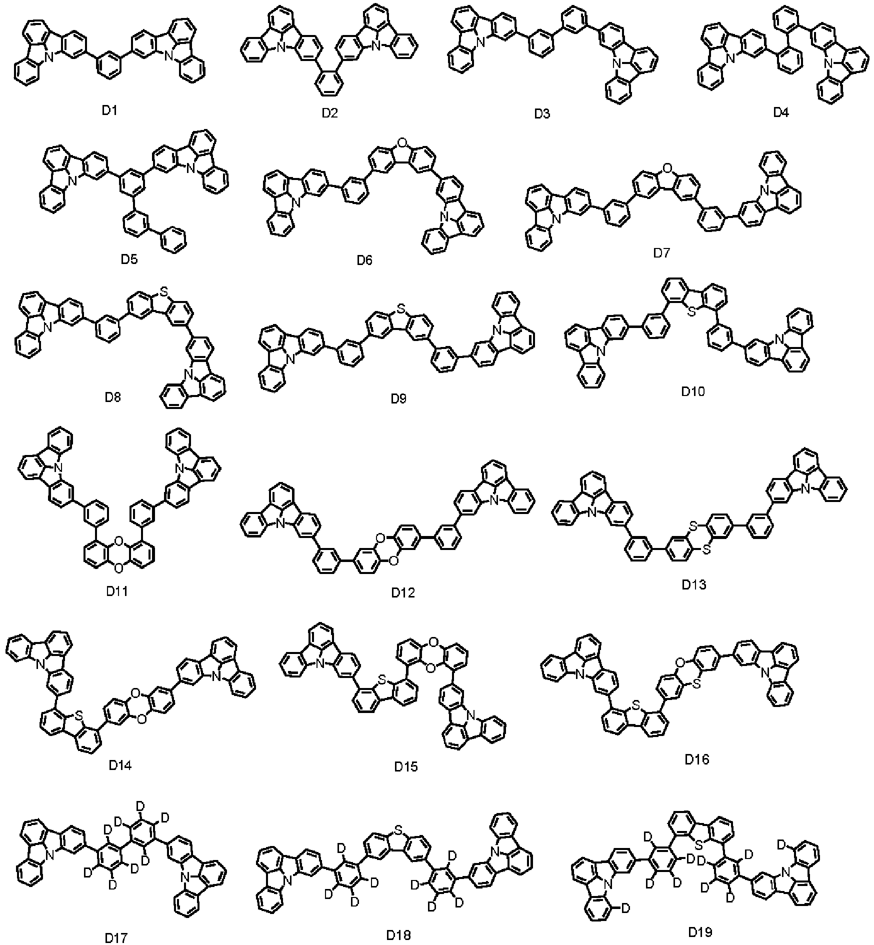

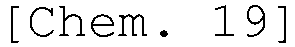

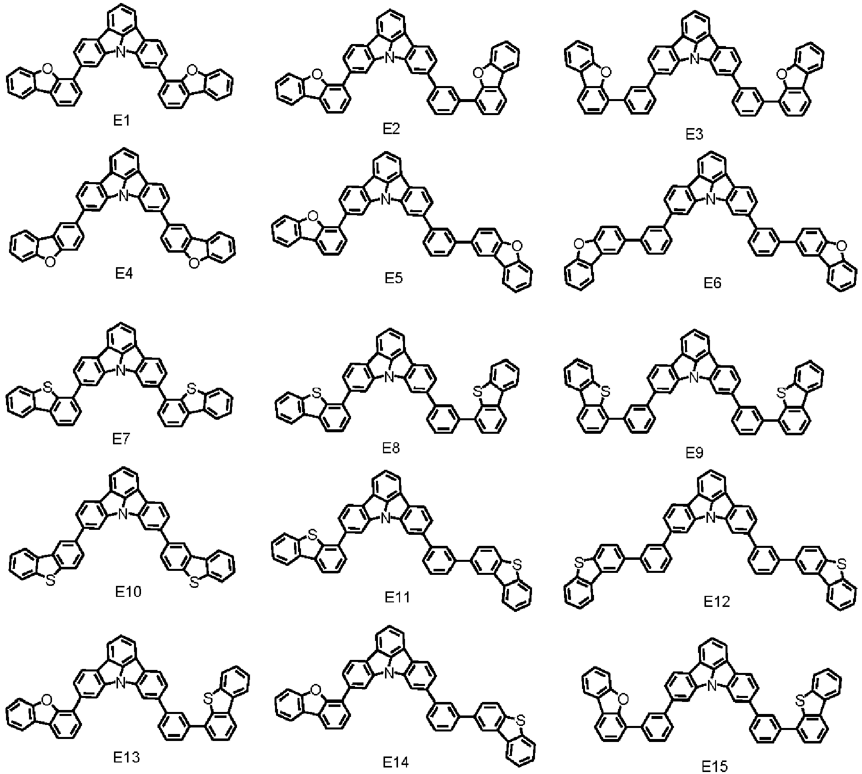

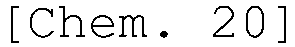

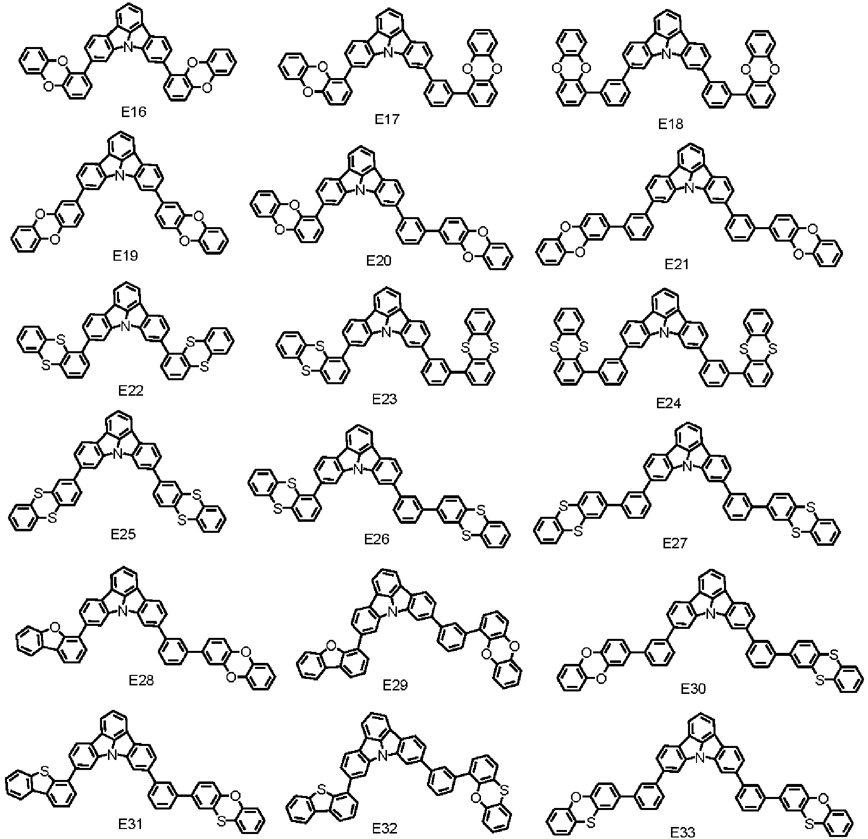

The Exemplified Compounds belonging to Group A are compounds of the formula [1] in which n = 0 and HAr₁ is the formula [b]. The compounds of Group A have a low molecular weight and a low sublimation temperature, and thus exhibit an effect of providing an increased margin of the sublimation temperature relative to the decomposition temperature.

The Exemplified Compounds belonging to Group B are compounds of the formula [1] in which n = 0 and HAr₁ is the formula [c]. The compounds of Group B have HAr₁ having a fused ring structure composed of three or more rings, and thus exhibit effects of having high thermal stability and high T₁.

The Exemplified Compounds belonging to Group C are compounds of the formula [1] in which n = 0 and HAr₁ is the formula [d], or compounds in which L₁ includes a residue of the compound represented by the formula [a]. The compounds of Group C have L₁-HAr₁ including a cyclic structure having an ether or thioether bridge and include a large number of unshared electron pairs of heteroatoms, and thus exhibit an effect of having a strong compatibility effect.

The Exemplified Compounds belonging to Group D are compounds represented by the formula [2]. The compounds of Group D have two indolocarbazole units and have high hole-transporting capability, and exhibit an effect of having a higher hole mobility.

The Exemplified Compounds belonging to Group E are compounds of the formula [1] in which n = 1. The compounds of Group E have high molecular weight and hence exhibit an effect of having improved thermal stability, and exhibit an effect of having a large permanent dipole moment.

### <<Organic light-emitting element>>

Hereinafter, an organic light-emitting element of the present embodiment will be described. The organic light-emitting element of the present embodiment includes at least a first electrode, a second electrode, and an organic compound layer disposed between the electrodes. One of the first electrode and the second electrode is an anode and the other is a cathode. In the organic light-emitting element of the present embodiment, the organic compound layer may be a single layer or may be a multilayer body composed of a plurality of layers as long as the organic compound layer includes a light-emitting layer. Here, when the organic compound layer is a multilayer body composed of a plurality of layers, the organic compound layer may include, in addition to the light-emitting layer, a hole injection layer, a hole transport layer, an electron blocking layer, a hole/exciton blocking layer, an electron transport layer, an electron injection layer, and the like. The light-emitting layer may be a single layer or may be a multilayer body composed of a plurality of layers.

In the organic light-emitting element of the present embodiment, at least one layer of the organic compound layer contains the organic compound according to the present embodiment. Specifically, the organic compound according to the present embodiment is contained in any of the above-described light-emitting layer, hole injection layer, hole transport layer, electron blocking layer, hole/exciton blocking layer, electron transport layer, electron injection layer, and the like. The organic compound according to the present embodiment is preferably contained in a hole transport layer, an electron blocking layer, a hole/exciton blocking layer, an electron transport layer, and a light-emitting layer. More preferably, the organic compound according to the present embodiment is contained in a light-emitting layer.

In the organic light-emitting element of the present embodiment, when the organic compound according to the present embodiment is contained in a light-emitting layer, the light-emitting layer may be a layer composed only of the organic compound according to the present embodiment, or may be a layer composed of the organic compound according to the present embodiment and another compound. Here, when the light-emitting layer is a layer composed of the organic compound according to the present embodiment and another compound, the organic compound according to the present embodiment may be used as the host of the light-emitting layer or may be used as the guest of the light-emitting layer. Alternatively, the organic compound according to the present embodiment may be used as an assist material that can be contained in the light-emitting layer. The organic compound of the present embodiment can be suitably used as a host of a light-emitting layer of a system using a triplet excited state for light emission, such as phosphorescence or delayed fluorescence. Thus, the light-emitting layer preferably further contains a phosphorescent light-emitting compound. The organic compound of the present embodiment can be used in mixture with an electron-transporting host. Thus, the light-emitting layer preferably further contains an electron-transporting compound. Here, the host is a compound having the highest mass ratio among compounds constituting the light-emitting layer. The guest is a compound having a lower mass ratio than the host among compounds constituting the light-emitting layer and is a compound responsible for main light emission. The assist material is a compound having a lower mass ratio than the host among compounds constituting the light-emitting layer and assisting light emission of the guest. Note that the assist material is also referred to as a second host. The host material can also be referred to as a first compound, and the assist material can also be referred to as a second compound.

The concentration of the host of the light-emitting layer according to the present embodiment relative to the entire light-emitting layer is preferably 10 mass% or more and 90 mass% or less, more preferably 20 mass% or more and 80 mass% or less, and still more preferably 30 mass% or more and 70 mass% or less. The concentration of the guest relative to the host based on the total amount of constituent materials of the light-emitting layer is 0.01 mass% or more and 50 mass% or less and is preferably 0.1 mass% or more and 20 mass% or less. From a viewpoint of suppressing concentration quenching, the concentration of the guest is particularly preferably 10 mass% or less.

The guest may be uniformly contained in the entirety of the layer in which the host serves as a matrix, or may be contained with a concentration gradient. Alternatively, the light-emitting layer may be formed such that the guest is partially contained in a specified region in the layer and the layer includes a region composed only of the host without containing the guest.

The light-emitting layer of the present embodiment may be a single layer or a multilayer, and can contain a light-emitting material having another emission color to cause color mixing. The multilayer means a state in which a light-emitting layer and another light-emitting layer are stacked. In this case, the emission color of the organic light-emitting element is not particularly limited. More specifically, the emission color may be white or may be an intermediate color. In the case of white, for example, when the emission color of the light-emitting layer is blue, another light-emitting layer emits a color different from blue, that is, green or red. Such films are formed by deposition or coating. A third light-emitting layer that emits blue light and a charge generation layer may be disposed between the light-emitting layer or the multilayered light-emitting layer and the first or second electrode in the present embodiment. The charge generation layer provides a function as a tandem element: electrons originated from the charge generation layer and holes injected from the first electrode undergo charge recombination to generate excitons, and holes originated from the charge generation layer and electrons injected from the second electrode undergo charge recombination to form excitons. Accordingly, internal quantum efficiency is doubled. At that time, the organic light-emitting element of the present embodiment can be applied to one side of the tandem element as a yellow light-emitting layer that provides a complementary color to blue light emission. Thus, a tandem element can be formed with a blue light-emitting layer, using a multilayered light-emitting layer composed of the light-emitting layer of the present embodiment, to thereby provide a white light-emitting element. The third light-emitting layer contains at least a third organic compound and a fourth organic compound. The third organic compound is a host material, and the fourth organic compound is a blue light-emitting material.

Specific examples of the element configuration of the organic light-emitting element of the present embodiment include multilayer element configurations in which electrode layers and organic compound layers described in the following (1) to (6) are sequentially stacked on substrates. Note that, in all the element configurations, the organic compound layers necessarily include a light-emitting layer containing a light-emitting material.
(1) Anode/light-emitting layer/cathode
(2) Anode/hole transport layer/light-emitting layer/electron transport layer/cathode
(3) Anode/hole transport layer/light-emitting layer/electron transport layer/electron injection layer/cathode
(4) Anode/hole injection layer/hole transport layer/light-emitting layer/electron transport layer/cathode
(5) Anode/hole injection layer/hole transport layer/light-emitting layer/electron transport layer/electron injection layer/cathode
(6) Anode/hole transport layer/electron blocking layer/light-emitting layer/hole blocking layer/electron transport layer/cathode

However, these element configuration examples are merely non-limiting very basic element configurations. For example, various layer configurations can be employed, such as a layer configuration in which an insulating layer, an adhesion layer, or an interference layer is disposed at an interface between an electrode and an organic compound layer, a layer configuration in which an electron transport layer or a hole transport layer is constituted by two layers having different ionization potentials, and a layer configuration in which a light-emitting layer is constituted by two layers of different light-emitting materials.

Of the above-described element configurations (1) to (6), the configuration (6) includes both an electron blocking layer and a hole blocking layer and hence is preferred. That is, in (6) including the electron blocking layer and the hole blocking layer, both carriers of holes and electrons can be reliably confined in the light-emitting layer, so that the organic light-emitting element has high luminous efficiency without carrier leakage. Here, in the organic light-emitting element of the present embodiment, in a first organic compound and a second organic compound constituting the light-emitting layer, all freely rotatable single bonds are carbon-carbon bonds, preferably bonds between sp² carbons. That is, the light-emitting layer is preferably formed of host materials having high planarity. As a result, hole-transporting capability and electron-transporting capability become higher than those of general organic light-emitting elements. Accordingly, the electron blocking layer and the hole blocking layer play an important role. For example, the hole blocking layer needs to be stable against holes and hence the compound of the hole blocking layer is preferably an organic compound having low reactivity, and more preferably an organic compound composed only of hydrocarbons. For example, the electron blocking layer also needs to be stable against electrons and hence the compound of the electron blocking layer is preferably an organic compound having low reactivity, and more preferably an organic compound in which all freely rotatable single bonds are carbon-carbon bonds and preferably bonds between sp² carbons.

The mode of extracting light output from the light-emitting layer (element form) may be a so-called bottom emission system in which light is extracted from an electrode on a substrate side, or may be a so-called top emission system in which light is extracted from a side opposite from the substrate. Alternatively, a both-side extraction system in which light is extracted from the substrate side and the side opposite from the substrate can be employed.

The organic compound according to the present embodiment can be used as a constituent material of an organic compound layer other than the light-emitting layer constituting the organic light-emitting element of the present embodiment. Specifically, the organic compound may be used as a constituent material of an electron transport layer, an electron injection layer, a hole transport layer, a hole injection layer, a hole blocking layer, or the like. In this case, the emission color of the organic light-emitting element is not particularly limited. More specifically, the emission color may be white or may be an intermediate color.

### <Other compounds>

In the organic light-emitting element according to the present embodiment, publicly known low-molecular-weight or high-molecular-weight hole-injecting compounds or hole-transporting compounds, compounds serving as hosts, light-emitting compounds, electron-injecting compounds or electron-transporting compounds, or the like can be additionally used as necessary. Examples of these compounds will be described below.

The hole-injecting/transporting materials are preferably materials having high hole mobility in order to facilitate injection of holes from the anode and to transport injected holes to the light-emitting layer. In addition, in order to suppress deterioration of film quality such as crystallization in the organic light-emitting element, the materials are preferably materials having a high glass transition temperature. Examples of low-molecular-weight or high-molecular-weight materials having hole-injecting/transporting performance include triarylamine derivatives, arylcarbazole derivatives, phenylenediamine derivatives, stilbene derivatives, phthalocyanine derivatives, porphyrin derivatives, poly(vinylcarbazole), poly(thiophene), and other conductive polymers. Such hole-injecting/transporting materials are also suitably used for the electron blocking layer. The following are non-limiting specific examples of compounds used as hole-injecting/transporting materials.

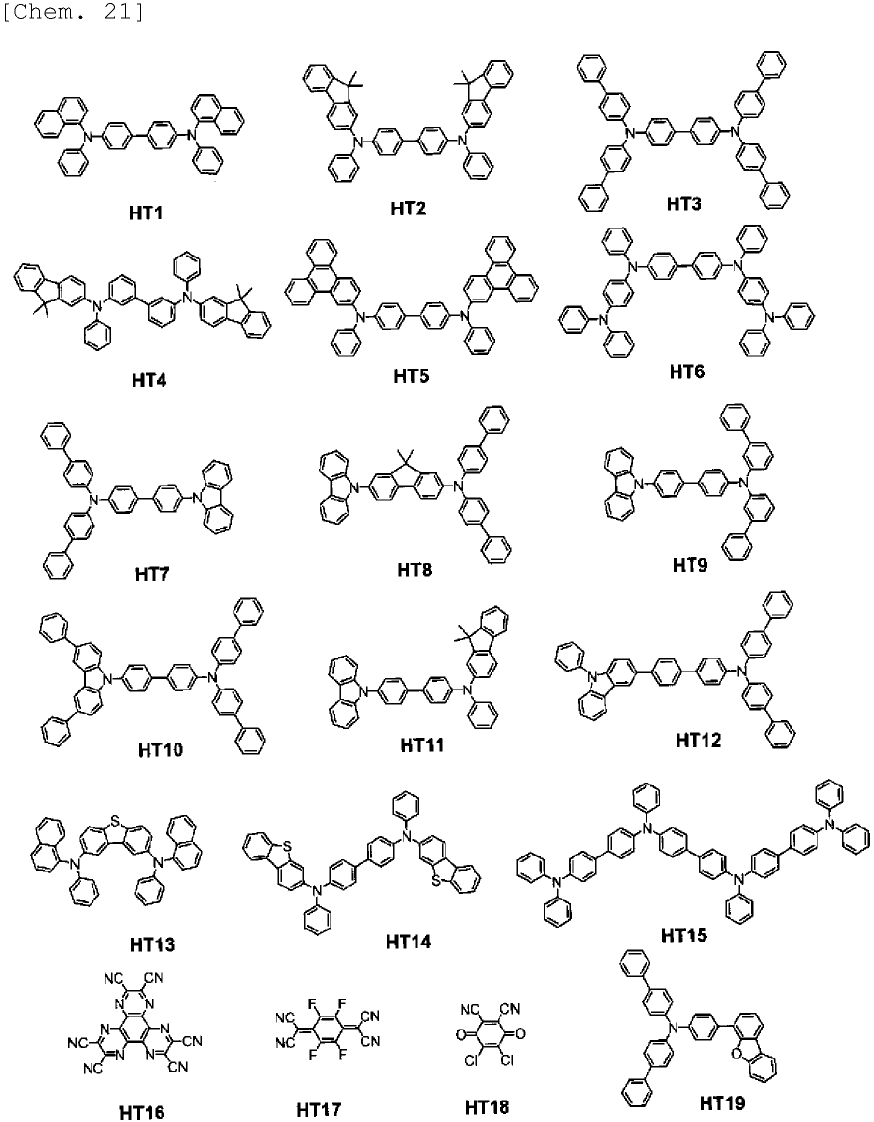

Examples of light-emitting materials mainly relating to the light-emitting function include fused ring compounds (for example, fluorene derivatives, naphthalene derivatives, pyrene derivatives, perylene derivatives, tetracene derivatives, anthracene derivatives, rubrene, and the like), quinacridone derivatives, coumarin derivatives, stilbene derivatives, organic aluminum complexes such as tris(8-quinolinolato)aluminum, iridium complexes, platinum complexes, rhenium complexes, copper complexes, europium complexes, ruthenium complexes, and polymer derivatives such as poly(phenylenevinylene) derivatives, poly(fluorene) derivatives, and poly(phenylene) derivatives. The following are non-limiting specific examples of compounds used as light-emitting materials.

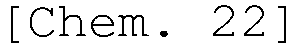

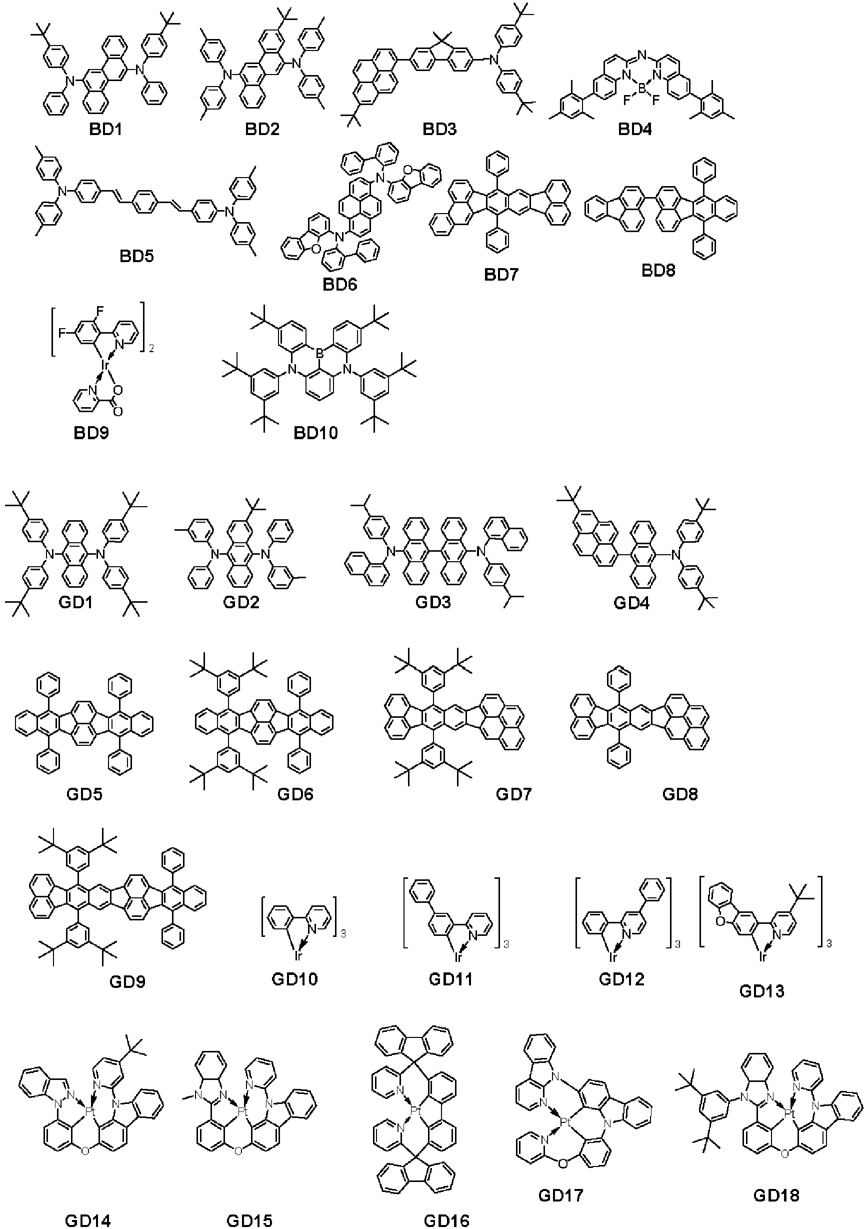

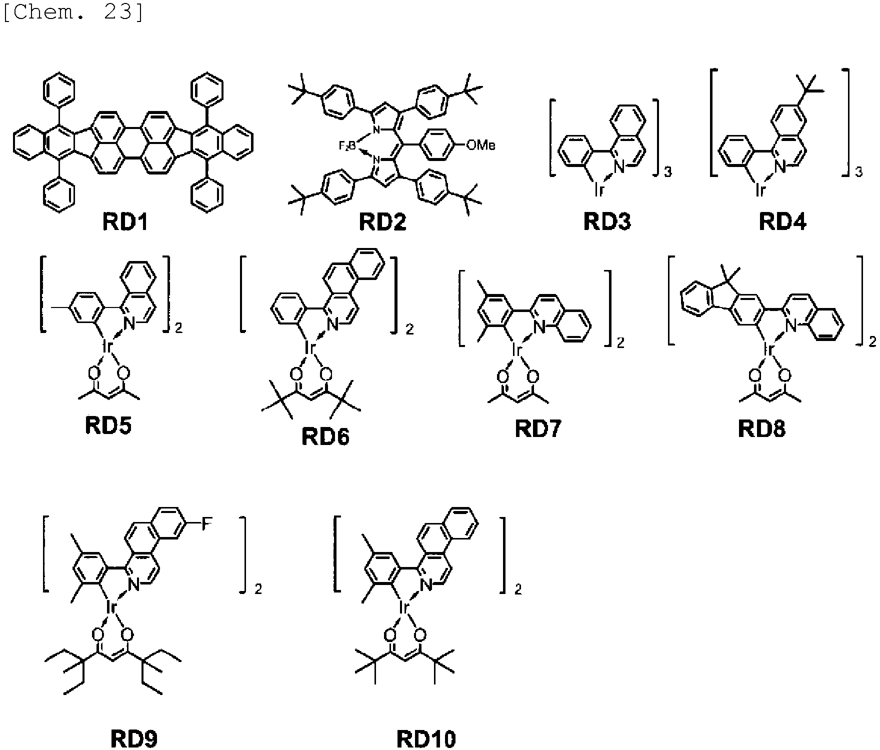

As a light-emitting-layer host or a light-emitting assist material contained in the light-emitting layer, a compound other than the organic compound of the present embodiment may be contained as a third component. Examples of the third component include aromatic hydrocarbon compounds or derivatives thereof, carbazole derivatives, azine derivatives, xanthone derivatives, dibenzofuran derivatives, dibenzothiophene derivatives, organic aluminum complexes such as tris(8-quinolinolato)aluminum, and organic beryllium complexes.

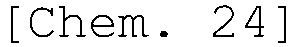

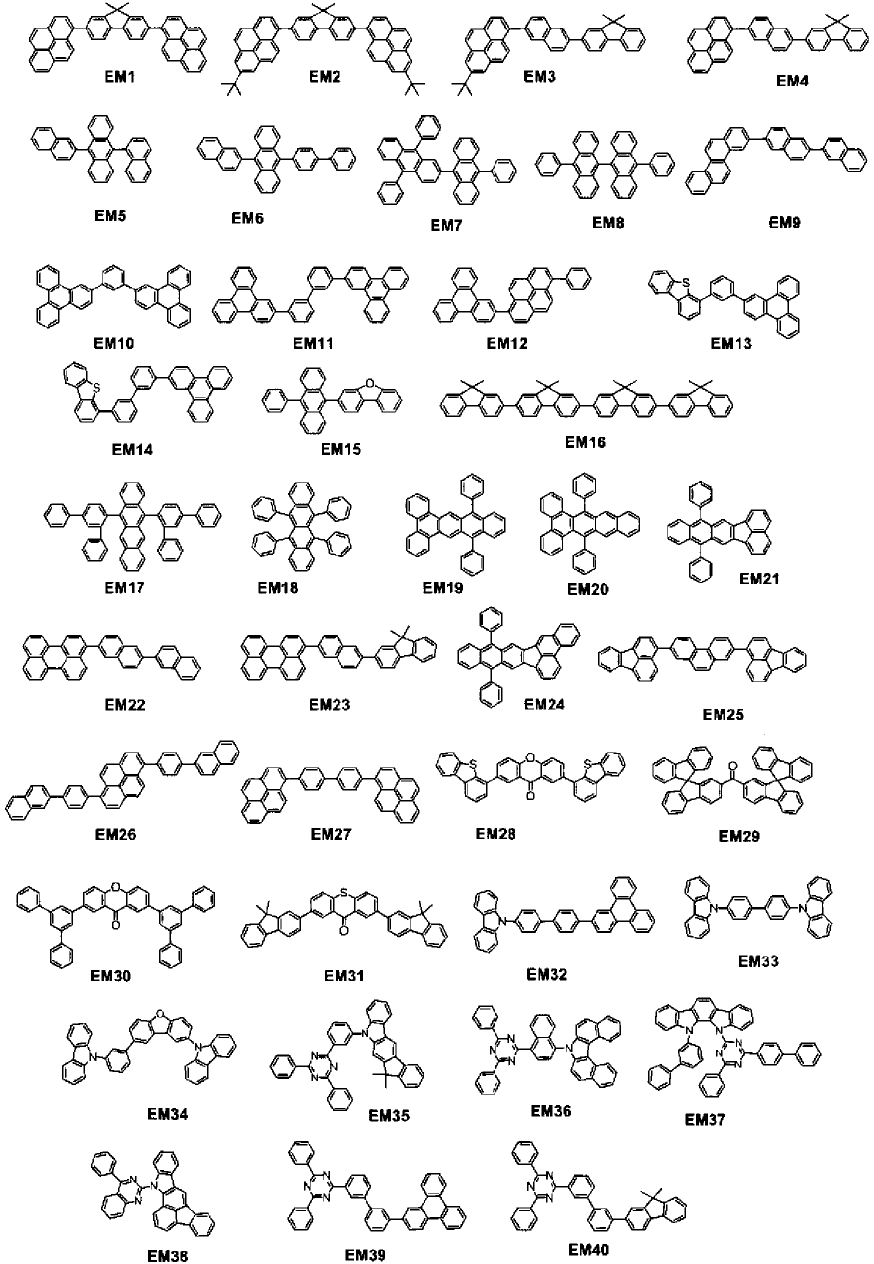

The electron-transporting material can be appropriately selected from materials that can transport electrons injected from the cathode to the light-emitting layer, and is selected in consideration of, for example, a balance with hole mobility of the hole-transporting material. Examples of the material having electron-transporting performance include oxadiazole derivatives, oxazole derivatives, pyrazine derivatives, triazole derivatives, triazine derivatives, quinoline derivatives, quinoxaline derivatives, phenanthroline derivatives, organic aluminum complexes, and fused ring compounds (for example, fluorene derivatives, naphthalene derivatives, chrysene derivatives, and anthracene derivatives). The electron-transporting material is also suitably used for the hole blocking layer. The following are non-limiting specific examples of compounds used as electron-transporting materials.

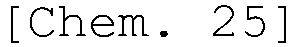

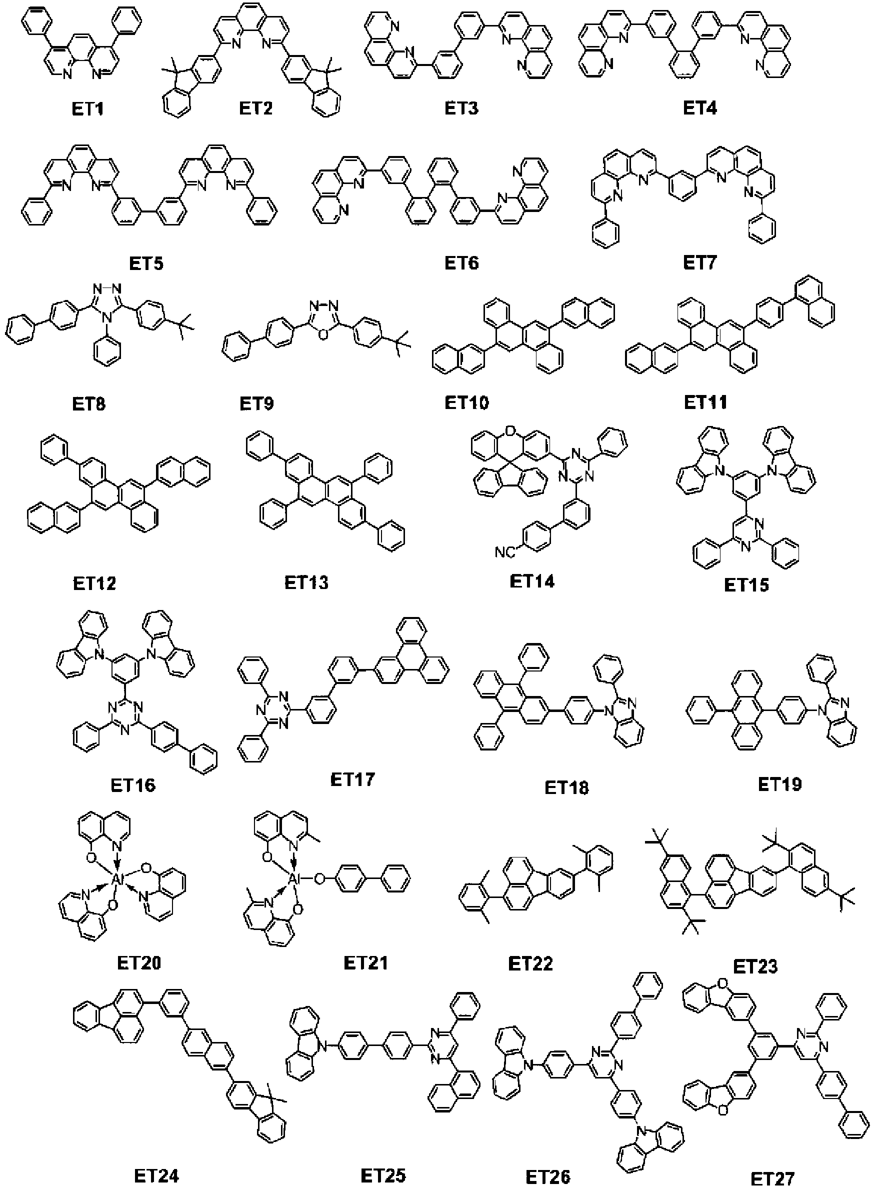

The electron-injecting material can be appropriately selected from materials that can easily inject electrons from the cathode, and is selected in consideration of, for example, a balance with hole-injecting performance. Examples of organic compounds include n-type dopants and reducing dopants. Examples include compounds containing an alkali metal such as lithium fluoride, lithium complexes such as lithium quinolinol, benzimidazolidene derivatives, imidazolidene derivatives, fulvalene derivatives, and acridine derivatives. The electron-injecting material can also be used together with the above electron-transporting material.

### <Configuration of organic light-emitting element>

The organic light-emitting element is provided by forming a first electrode, an organic compound layer, and a second electrode on a substrate. An insulating layer may be provided on the substrate. A protective layer, a color filter, a microlens, and the like may be provided on the second electrode. When a color filter is provided, a planarization layer may be provided between the color filter and the protective layer. The planarization layer can be formed of an acrylic resin or the like. The same applies to a case where a planarization layer is provided between the color filter and the microlens. One of the first electrode and the second electrode may be an anode, and the other may be a cathode.

### [Substrate]

The substrate may be formed of quartz, glass, a silicon wafer, resin, metal, or the like. On the substrate, a switching element such as a transistor and wiring may be provided, and an insulating layer may be provided thereon. The material of the insulating layer is not limited as long as a contact hole can be formed such that wiring to the first electrode can be formed and insulation from wiring not connected can be ensured. Examples include resins such as polyimide, silicon oxide, and silicon nitride.

### [Electrodes]

A pair of electrodes can be used as the electrodes. The pair of electrodes can be an anode and a cathode. When an electric field is applied in a direction in which the organic light-emitting element emits light, the electrode having a higher potential is the anode, and the other is the cathode. In other words, the electrode supplying holes to the light-emitting layer is the anode, and the electrode supplying electrons to the light-emitting layer is the cathode.

The constituent material of the anode is preferably a material having a work function as high as possible. Examples thereof include elemental metals such as gold, platinum, silver, copper, nickel, palladium, cobalt, selenium, vanadium, and tungsten, mixtures containing the foregoing, alloys that are combinations of the foregoing, and metal oxides such as tin oxide, zinc oxide, indium oxide, indium tin oxide (ITO), and indium zinc oxide. Other examples include conductive polymers such as polyaniline, polypyrrole, and polythiophene.

These electrode substances may be used alone or may be used in combination of two or more thereof. The anode may be constituted by a single layer or may be constituted by a plurality of layers.

In the case of using a reflective electrode, it can be formed of, for example, chromium, aluminum, silver, titanium, tungsten, molybdenum, an alloy of the foregoing, or a stacked structure of the foregoing. Alternatively, such a material can be used to form a reflective film having no role as an electrode. In the case of using a transparent electrode, non-limiting examples include an oxide transparent conductive layer of indium tin oxide (ITO), indium zinc oxide, or the like. The electrode can be formed by photolithography.

On the other hand, the constituent material of the cathode is preferably a material having a low work function. Examples thereof include alkali metals such as lithium, alkaline earth metals such as calcium, elemental metals such as aluminum, titanium, manganese, silver, lead, and chromium, and mixtures containing the foregoing. Alternatively, alloys that are combinations of the foregoing elemental metals can be used. Examples thereof include magnesium-silver, aluminum-lithium, aluminum-magnesium, silver-copper, and zinc-silver. Metal oxides such as indium tin oxide (ITO) can also be used. These electrode substances may be used alone or may be used in combination of two or more thereof. The cathode may have a single-layer configuration or may have a multilayer configuration. In particular, silver is preferably used, and a silver alloy is more preferably used in order to reduce aggregation of silver. As long as aggregation of silver can be reduced, the ratio of the alloy is not limited. For example, silver:other metal may be 1:1, 3:1, or the like.

The cathode is not particularly limited and may be formed as an oxide conductive layer of ITO or the like to provide a top emission element, or may be formed as a reflective electrode of aluminum (Al) or the like to provide a bottom emission element. The method for forming the cathode is not particularly limited, but is more preferably a method such as DC or AC sputtering because the film has high coverage and tends to have reduced resistance.

### [Organic compound layer]

The organic compound layer may be formed as a single layer or may be formed as a plurality of layers. When the organic compound layer includes a plurality of layers, the layers may be referred to as, depending on functions thereof, a hole injection layer, a hole transport layer, an electron blocking layer, a light-emitting layer, a hole blocking layer, an electron transport layer, or an electron injection layer. The organic compound layer is mainly formed of an organic compound, but may contain inorganic atoms or an inorganic compound. For example, the organic compound layer may contain copper, lithium, magnesium, aluminum, iridium, platinum, molybdenum, zinc, or the like. The organic compound layer may be disposed between the first electrode and the second electrode, and may be disposed in contact with the first electrode and the second electrode.

Organic compound layers (a hole injection layer, a hole transport layer, an electron blocking layer, a light-emitting layer, a hole blocking layer, an electron transport layer, an electron injection layer, and the like) constituting an organic light-emitting element according to an embodiment of the present invention are formed by the following methods.

The organic compound layers constituting the organic light-emitting element according to an embodiment of the present invention can be formed by a dry process such as a vacuum deposition method, an ionized deposition method, sputtering, or plasma. In place of the dry process, a wet process can also be used in which a layer is formed by dissolving in an appropriate solvent and performing a publicly known coating method (for example, a spin coating method, a casting method, a microgravure coating method, a gravure coating method, a bar coating method, a roll coating method, a wire bar coating method, a dip coating method, a spray coating method, a screen printing method, a flexographic printing method, an offset printing method, an inkjet printing method, a capillary coating method, a nozzle coating method, an LB method, or the like). In particular, a vacuum deposition method, an ionized deposition method, an inkjet printing method, a nozzle coating method, and the like are suitable for manufacturing large-area organic light-emitting elements.

In such a case of forming a layer by a vacuum deposition method, a solution coating method, or the like, crystallization and the like are less likely to occur and high temporal stability is provided. In the case of forming a film by a coating method, the film can also be formed in combination with an appropriate binder resin.

Non-limiting examples of the binder resin include polyvinylcarbazole resins, polycarbonate resins, polyester resins, ABS resins, acrylic resins, polyimide resins, phenol resins, epoxy resins, silicone resins, and urea resins.

Such binder resins may be used alone as a homopolymer or a copolymer, or in combination of two or more thereof. Furthermore, as necessary, publicly known additives such as a plasticizer, an antioxidant, and an ultraviolet absorber may be used in combination.

The thickness of each layer in the organic light-emitting element is preferably, in general, 1 nm or more and 10 µm or less. In particular, the film thickness of the light-emitting layer of the organic compound layer is preferably 10 nm or more and 100 nm or less in order to obtain effective light-emitting characteristics.

### [Protective layer]

A protective layer may be provided on the second electrode. For example, glass provided with a moisture absorbent can be bonded onto the second electrode, to thereby reduce intrusion of water and the like into the organic compound layer to reduce occurrence of display defects. In another embodiment, a passivation film of silicon nitride or the like may be provided on the second electrode to reduce intrusion of water and the like into the organic compound layer. For example, the second electrode is formed, and may then be transferred to another chamber without breaking vacuum, and a silicon nitride film having a thickness of 2 µm may be formed by a CVD method as the protective layer. After film formation by the CVD method, a protective layer formed using an atomic layer deposition method (ALD method) may be provided. The material of the film formed by the ALD method is not limited, and may be silicon nitride, silicon oxide, aluminum oxide, or the like. Silicon nitride may further be formed by the CVD method on the film formed by the ALD method. The film formed by the ALD method may have a smaller film thickness than the film formed by the CVD method. Specifically, the film thickness may be 50% or less, and further may be 10% or less.

### [Color filter]

A color filter may be provided on the protective layer. For example, a color filter in consideration of the size of the organic light-emitting element may be provided on another substrate, and it may be bonded to a substrate provided with the organic light-emitting element, or the color filter may be patterned on the above-described protective layer by photolithography. The color filter may be formed of a polymer.

### [Planarization layer]

A planarization layer may be provided between the color filter and the protective layer. The planarization layer is provided for the purpose of reducing irregularities of the underlying layer. The planarization layer may also be referred to as a material resin layer in order not to limit the purpose. The planarization layer may be formed of an organic compound, and may be formed of a low-molecular-weight compound or a high-molecular-weight compound, but is preferably formed of a high-molecular-weight compound.

The planarization layer may be provided over and under the color filter, and the constituent materials thereof may be the same or different. Specific examples include polyvinylcarbazole resins, polycarbonate resins, polyester resins, ABS resins, acrylic resins, polyimide resins, phenol resins, epoxy resins, silicone resins, and urea resins.

### [Microlens]

The organic light-emitting element may include an optical member such as a microlens on the light emission side thereof. The microlens can be formed of an acrylic resin, an epoxy resin, or the like. The microlens may be provided for the purpose of increasing the amount of light extracted from the organic light-emitting element and controlling the direction of the extracted light. The microlens may have a hemispherical shape. In the case of having a hemispherical shape, among tangents in contact with the hemisphere, a tangent parallel to the insulating layer is present, and the contact point between the tangent and the hemisphere is the apex of the microlens. The apex of the microlens can be similarly determined in any sectional view. That is, among tangents in contact with the semicircle of the microlens in the sectional view, a tangent parallel to the insulating layer is present, and the contact point between the tangent and the semicircle is the apex of the microlens.

The midpoint of the microlens can also be defined. In a section of the microlens, a line segment is assumed from a point where the arc shape ends to another point where the arc shape ends, and the midpoint of the line segment can be referred to as the midpoint of the microlens. The section where the apex and the midpoint are determined may be a section perpendicular to the insulating layer.

### [Opposing substrate]

An opposing substrate may be provided on the planarization layer. The opposing substrate is provided at a position corresponding to the above-described substrate and hence is referred to as the opposing substrate. The constituent material of the opposing substrate may be the same as that of the above-described substrate. When the above-described substrate is defined as a first substrate, the opposing substrate may be defined as a second substrate.

### [Pixel circuit]

An organic light-emitting apparatus including the organic light-emitting element may include a pixel circuit connected to the organic light-emitting element. The pixel circuit may be an active-matrix type that independently controls light emission of a first light-emitting element and a second light-emitting element. The active-matrix-type circuit may employ voltage programming or current programming. The driving circuit includes a pixel circuit for each pixel. The pixel circuit may include a light-emitting element, a transistor for controlling light emission luminance of the light-emitting element, a transistor for controlling light emission timing, a capacitor for holding the gate voltage of the transistor for controlling light emission luminance, and a transistor for connecting to GND without passing through the light-emitting element.

The light-emitting apparatus includes a display region and a peripheral region disposed around the display region. The display region includes pixel circuits, and the peripheral region includes a display control circuit. Mobility of transistors constituting the pixel circuits may be lower than mobility of transistors constituting the display control circuit. The slope of current-voltage characteristics of the transistors constituting the pixel circuits may be smaller than the slope of current-voltage characteristics of the transistors constituting the display control circuit. The slope of current-voltage characteristics can be measured from the so-called Vg-Ig characteristics. The transistors constituting the pixel circuits are transistors connected to light-emitting elements such as the first light-emitting element.

### [Pixels]

An organic light-emitting apparatus including the organic light-emitting element may include a plurality of pixels. Each pixel includes sub-pixels that emit mutually different colors. The sub-pixels may have, for example, RGB emission colors.

In a pixel, a region also referred to as a pixel aperture emits light. This region is the same as the first region. The pixel aperture may be 15 µm or less and may be 5 µm or more. More specifically, the pixel aperture may be 11 µm, 9.5 µm, 7.4 µm, 6.4 µm, or the like. The distance between sub-pixels may be 10 µm or less, and specifically may be 8 µm, 7.4 µm, or 6.4 µm.

Pixels can take publicly known arrangement forms in a plan view. For example, a stripe arrangement, a delta arrangement, a PenTile arrangement, or a Bayer arrangement may be employed. The shape of each sub-pixel in a plan view may be any publicly known shape. For example, a quadrangle such as a rectangle or a rhombus, or a hexagon may be employed. Of course, a shape that is not an exact figure but is close to a rectangle is encompassed in the rectangle. The sub-pixel shape and the pixel arrangement can be used in combination.

### <Applications of organic light-emitting element>

The organic light-emitting element according to the present embodiment can be used as a constituent member of a display device or a lighting apparatus. Other applications include an exposure light source of an electrophotographic image forming apparatus, a backlight of a liquid crystal display device, and a light-emitting apparatus including a color filter on a white light source.

The display device may be an image information processing apparatus that includes an image input unit where image information from an area CCD, a linear CCD, a memory card, or the like is input, includes an information processing unit that processes the input information, and displays the input image in a display unit. The display device includes a plurality of pixels, and at least one of the plurality of pixels may include the organic light-emitting element of the present embodiment and an active element such as a transistor connected to the organic light-emitting element. In this case, the substrate may be a semiconductor substrate of silicon or the like, and the transistor may be a MOSFET formed in the substrate. An image display device includes an input unit where image information is input and a display unit configured to output an image, and the display unit includes the display device of the present embodiment.

A display unit included in an image pickup device or an inkjet printer may have a touch panel function. The driving method of the touch panel function may be an infrared method, a capacitive method, a resistive film method, or an electromagnetic induction method, and is not particularly limited. The display device may also be used for a display unit of a multifunction printer.

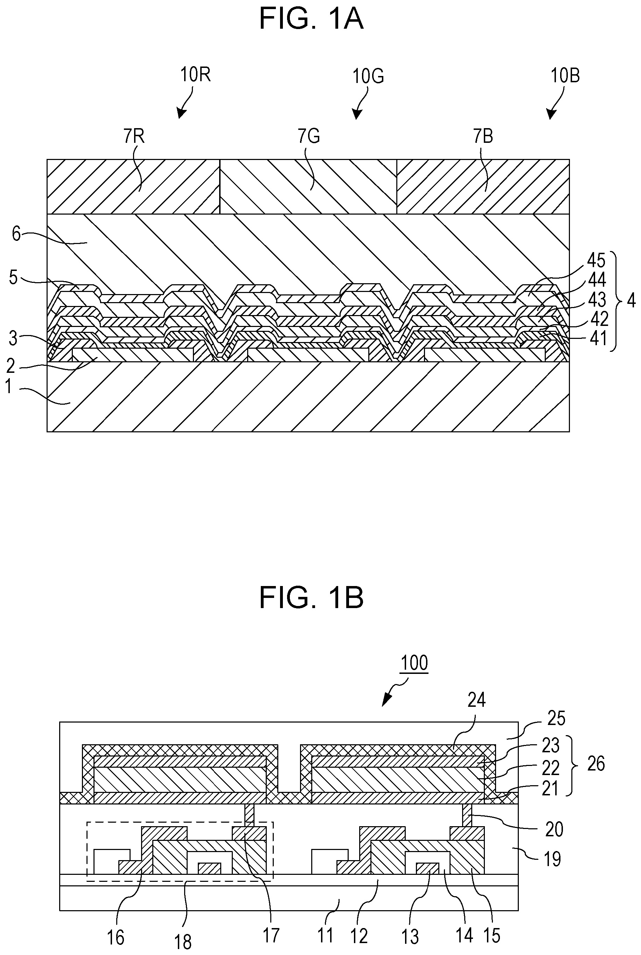

Hereinafter, a display device according to the present embodiment will be described with reference to drawings. Fig. 1A and Fig. 1B are schematic sectional views illustrating an example of a display device including an organic light-emitting element and a transistor connected to the organic light-emitting element. The transistor is an example of an active element. The transistor may be a thin film transistor (TFT).

Fig. 1A is a schematic sectional view illustrating an example of a pixel, which is a constituent element of the display device according to the present embodiment. The pixel includes sub-pixels 10. The sub-pixels are divided into 10R, 10G, and 10B according to light emission thereof. The emission color may be distinguished by the emission wavelength from the light-emitting layer, or light emitted from the sub-pixel may be selectively transmitted or color-converted by a color filter or the like. Each sub-pixel 10 includes, on an interlayer insulating layer 1, a reflective electrode serving as a first electrode 2, an insulating layer 3 covering the edge of the first electrode 2, an organic compound layer 4 covering the first electrode 2 and the insulating layer 3, a transparent electrode serving as a second electrode 5, a protective layer 6, and a color filter 7.

A transistor and a capacitive element may be disposed in the underlying layer or inside of the interlayer insulating layer 1. The transistor and the first electrode 2 may be electrically connected via a contact hole or the like (not shown).

The insulating layer 3 is also referred to as a bank or a pixel isolation film. The insulating layer 3 covers the edge of the first electrode 2 and is disposed so as to surround the first electrode 2. A portion where the insulating layer 3 is not disposed is in contact with the organic compound layer 4 and serves as a light-emitting region.

The organic compound layer 4 includes a hole injection layer 41, a hole transport layer 42, a light-emitting layer 43, a hole blocking layer 44, and an electron transport layer 45.

The second electrode 5 may be a transparent electrode, a reflective electrode, or a semi-transmissive electrode.

The protective layer 6 reduces penetration of moisture into the organic compound layer 4. The protective layer 6 is illustrated as a single layer, but may be a plurality of layers. Each layer may be an inorganic compound layer or an organic compound layer.

The color filter 7 is divided into 7R, 7G, and 7B according to color. The color filter 7 may be formed on a planarization film (not shown). The color filter 7 may underlie a resin protective layer (not shown). The color filter 7 may be formed on the protective layer 6. Alternatively, the color filter 7 may be provided on an opposing substrate such as a glass substrate and then bonded.

A display device 100 of Fig. 1B includes an organic light-emitting element 26 and a TFT 18, which is an example of a transistor. A substrate 11 of glass, silicon, or the like is provided, and an insulating layer 12 is provided thereon. Active elements such as the TFT 18 are arranged on the insulating layer 12, and a gate electrode 13, a gate insulating film 14, and a semiconductor layer 15 of the active element are provided. The TFT 18 includes a drain electrode 16 and a source electrode 17. An insulating film 19 is provided over the TFT 18. An anode 21 constituting the organic light-emitting element 26 and the source electrode 17 are connected via a contact hole 20 provided in the insulating film 19.

Note that the method of electrical connection between electrodes (the anode 21 and a cathode 23) included in the organic light-emitting element 26 and electrodes (the source electrode 17 and the drain electrode 16) included in the TFT 18 is not limited to the form illustrated in Fig. 1B. That is, it is sufficient that one of the anode 21 and the cathode 23 is electrically connected to one of the source electrode 17 and the drain electrode 16 of the TFT 18.

Fig. 1B illustrates the display device 100 such that an organic compound layer 22 is a single layer, but the organic compound layer 22 may be a plurality of layers. On the cathode 23, a first protective layer 24 and a second protective layer 25 for reducing deterioration of the organic light-emitting element 26 are provided.

In the display device 100 of Fig. 1B, a transistor is used as a switching element, but another switching element such as an MIM element may be used instead.

The transistor used in the display device 100 of Fig. 1B is not limited to a thin film transistor having an active layer on an insulating surface of a substrate, and may be a transistor using a single-crystal silicon wafer. Examples of the active layer include single-crystal silicon, non-single-crystal silicon such as amorphous silicon or microcrystalline silicon, and non-single-crystal oxide semiconductors such as indium zinc oxide and indium gallium zinc oxide. Note that the thin film transistor is also referred to as a TFT element.

The transistor included in the display device 100 of Fig. 1B may be formed in the substrate such as a Si substrate. This phrase "formed in the substrate" means that the substrate itself such as the Si substrate is processed to fabricate the transistor. That is, the substrate including a transistor therein can also be regarded as the substrate and the transistor that are formed as a single body.

In the organic light-emitting element according to the present embodiment, light emission luminance is controlled by a TFT, which is an example of a switching element; and a plurality of such organic light-emitting elements are disposed in a surface and their light emission luminances can be used to display an image. Note that the switching element according to the present embodiment is not limited to the TFT, and may be a transistor formed of low-temperature polysilicon, or an active matrix driver formed on a substrate such as a Si substrate. The phrase "on a substrate" can also mean "in the substrate". Whether a transistor is provided in the substrate or a TFT is used is selected depending on the size of the display unit; for example, in the case of a size of about 0.5 inches, the organic light-emitting element is preferably provided on a Si substrate.

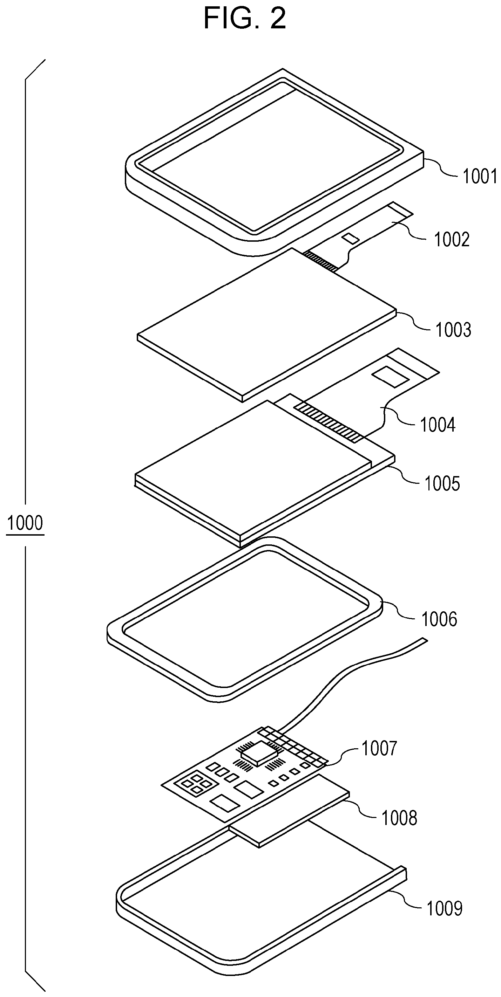

Fig. 2 is a schematic view illustrating an example of a display device according to the present embodiment. A display device 1000 may include, between an upper cover 1001 and a lower cover 1009, a touch panel 1003, a display panel 1005, a frame 1006, a circuit board 1007, and a battery 1008. A flexible printed circuit FPC 1002 and an FPC 1004 are respectively connected to the touch panel 1003 and the display panel 1005. Transistors are printed on the circuit board 1007. The battery 1008 may be omitted when the display device is not a portable device; even when the display device is a portable device, the battery 1008 may be provided at another position.

The display device according to the present embodiment may include color filters having red, green, and blue. The color filters having red, green, and blue may be arranged in a delta arrangement.

The display device according to the present embodiment may be used for a display unit of a portable terminal. In that case, the display device may have both a display function and an operation function. Examples of the portable terminal include cellular phones such as smartphones, tablets, and head-mounted displays.

The display device according to the present embodiment may be used for a display unit of an image pickup device including an optical unit including a plurality of lenses, and an image pickup element configured to receive light that has passed through the optical unit. The image pickup device may include a display unit configured to display information acquired by the image pickup element. The display unit may be a display unit exposed to the outside of the image pickup device or may be a display unit disposed in a finder. The image pickup device may be a digital camera or a digital video camera.

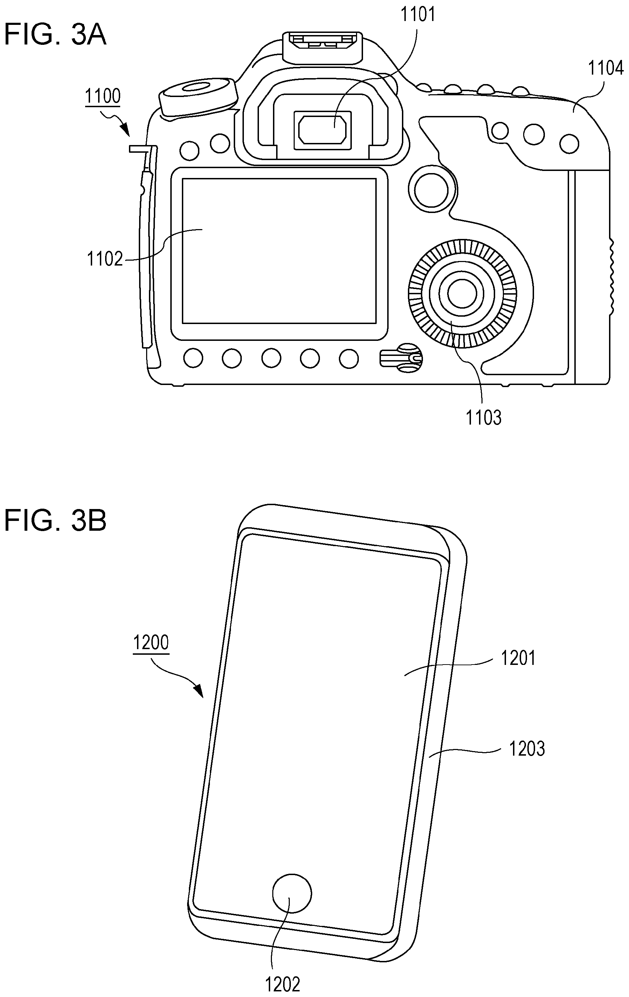

Fig. 3A is a schematic view illustrating an example of an image pickup device according to the present embodiment. An image pickup device 1100 may include a viewfinder 1101, a rear display 1102, an operation unit 1103, and a housing 1104. The viewfinder 1101 may include the display device according to the present embodiment. In that case, the display device may display not only an image to be captured but also environmental information, an image pickup instruction, and the like. The environmental information may include intensity of external light, direction of external light, the moving speed of the subject, a possibility that the subject is shielded by an obstruction, and the like.

The timing suitable for image pickup is a short time, and hence information is preferably displayed as quickly as possible. Thus, a display device using the organic light-emitting element of the present embodiment is preferably used. This is because the organic light-emitting element has a high response speed. Display devices using an organic light-emitting element are required to have a display speed, and such devices can be used more suitably than liquid crystal display devices.

The image pickup device 1100 includes an optical unit (not shown). The optical unit includes a plurality of lenses and forms an image on an image pickup element housed in the housing 1104. The relative positions of the plurality of lenses can be adjusted to thereby adjust focus. This operation can also be performed automatically. The image pickup device may also be referred to as a photoelectric conversion apparatus. The photoelectric conversion apparatus can include, as image pickup methods that do not sequentially capture images, a method of detecting a difference from a previous image, a method of cutting out from an image that is always recorded, and the like.

Fig. 3B is a schematic view illustrating an example of an electronic apparatus according to the present embodiment. An electronic apparatus 1200 includes a display unit 1201, an operation unit 1202, and a housing 1203. The housing 1203 may include a circuit, a printed board including the circuit, a battery, and a communication unit. The operation unit 1202 may be a button, or may be a touch-panel-based input unit. The operation unit 1202 may be a biometric recognition unit that recognizes a fingerprint to perform unlocking or the like. An electronic apparatus including a communication unit can also be referred to as a communication apparatus. The electronic apparatus 1200 may include a lens and an image pickup element to thereby further have a camera function. The image captured by the camera function is displayed on the display unit 1201. Examples of the electronic apparatus 1200 include smartphones and notebook computers.

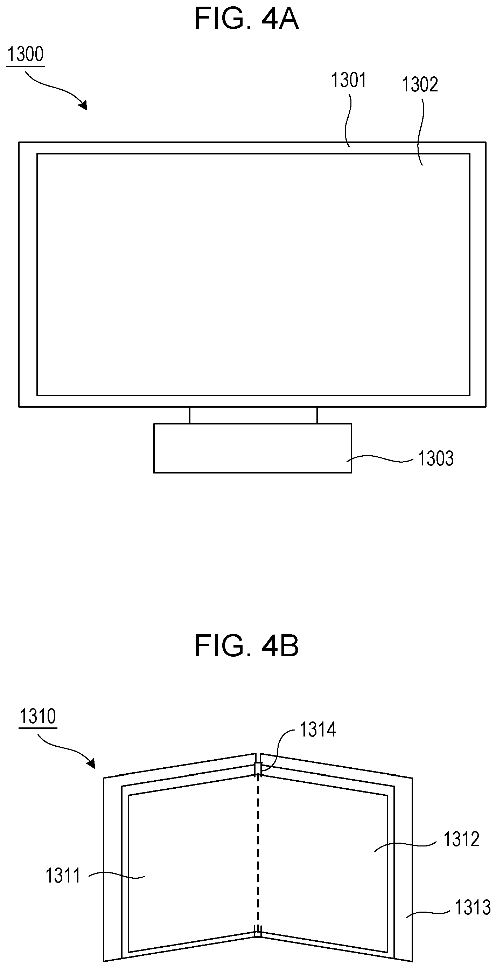

Fig. 4A and Fig. 4B are schematic views illustrating examples of a display device according to the present embodiment. Fig. 4A illustrates a display device such as a television monitor or a PC monitor. A display device 1300 includes a frame 1301 and a display unit 1302. The display unit 1302 may use the light-emitting element according to the present embodiment. The display device 1300 includes the frame 1301 and a base 1303 that supports the display unit 1302. The base 1303 is not limited to the form of Fig. 4A. The lower side of the frame 1301 may also serve as the base. The frame 1301 and the display unit 1302 may be curved. The radius of curvature thereof may be 5000 mm or more and 6000 mm or less.

Fig. 4B is a schematic view illustrating another example of the display device according to the present embodiment. A display device 1310 of Fig. 4B is configured to be foldable and is a so-called foldable display device. The display device 1310 includes a first display unit 1311, a second display unit 1312, a housing 1313, and a flexure point 1314. The first display unit 1311 and the second display unit 1312 may include the light-emitting element according to the present embodiment. The first display unit 1311 and the second display unit 1312 may constitute a single seamless display device. The first display unit 1311 and the second display unit 1312 can be divided at the flexure point. The first display unit 1311 and the second display unit 1312 may individually display different images, or the first and second display units may together display a single image.

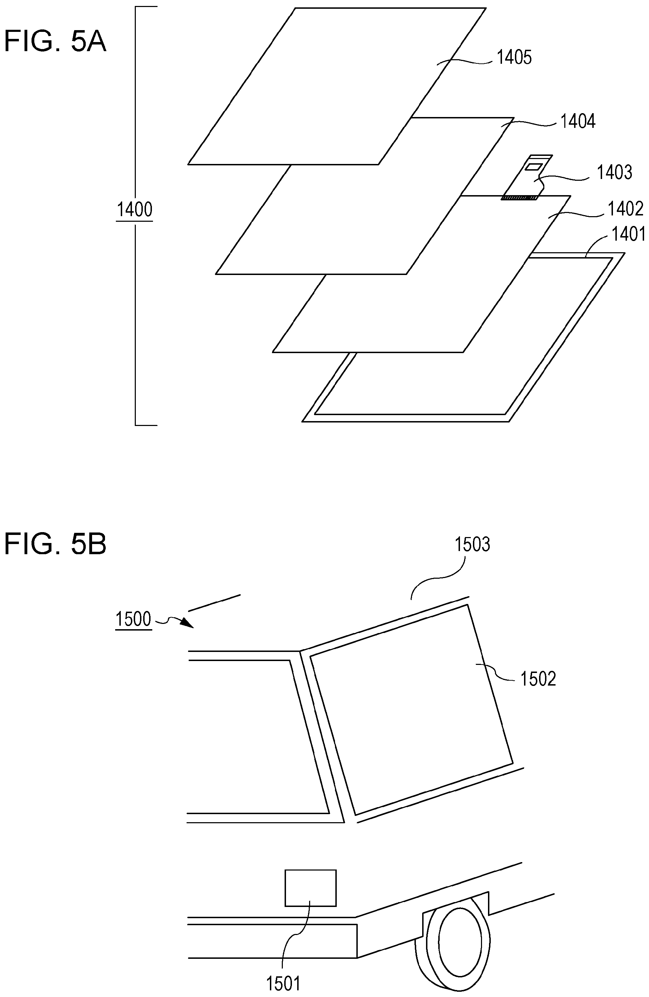

Fig. 5A is a schematic view illustrating an example of a lighting apparatus according to the present embodiment. A lighting apparatus 1400 may include a housing 1401, a light source 1402, a circuit board 1403, and an optical filter 1404 and a light diffusion unit 1405 that transmit light emitted from the light source 1402. The light source 1402 may include the organic light-emitting element according to the present embodiment. The optical filter 1404 may be a filter that improves color rendering of the light source. The light diffusion unit 1405 can effectively diffuse light from the light source, such as for light-up, and can deliver light to a wide range. The optical filter 1404 and the light diffusion unit 1405 may be provided on the light output side of the lighting. As necessary, a cover may be provided on the outermost side.

The lighting apparatus is, for example, an apparatus that illuminates an indoor space. The lighting apparatus may emit white, neutral white, or any other color from blue to red. The lighting apparatus may include a dimming circuit that adjusts the emission and a color adjustment circuit that adjusts the emission color. The lighting apparatus may include the organic light-emitting element of the present embodiment and a power supply circuit connected thereto. The power supply circuit is a circuit that converts an AC voltage into a DC voltage. The lighting apparatus may include an inverter circuit. The term "white" corresponds to a color temperature of 4200 K, and the term "neutral white" corresponds to a color temperature of 5000 K. The lighting apparatus may include a color filter.

The lighting apparatus according to the present embodiment may include a heat dissipation unit. The heat dissipation unit discharges heat in the apparatus to the outside of the apparatus, and may be formed of a metal having a high specific heat or liquid silicone.

Fig. 5B is a schematic view of an automobile serving as an example of a mobile object according to the present embodiment. The automobile includes a tail lamp serving as an example of a lighting device. An automobile 1500 includes a tail lamp 1501, and may be configured such that the tail lamp lights when a brake operation or the like is performed.

The tail lamp 1501 may include the organic light-emitting element according to the present embodiment. The tail lamp 1501 may include a protective member that protects the organic light-emitting element. The protective member may be formed of any material as long as it has a certain high strength and is transparent, but is preferably formed of polycarbonate or the like. The polycarbonate may be mixed with a furandicarboxylic acid derivative, an acrylonitrile derivative, or the like.

The automobile 1500 may include a vehicle body 1503 and a window 1502 attached thereto. When the window 1502 is not a window for checking the front and rear of the automobile, it may be a transparent display. The transparent display may include the organic light-emitting element according to the present embodiment. In this case, constituent materials such as electrodes included in the organic light-emitting element are composed of transparent members.

The mobile object according to the present embodiment may be a ship, an aircraft, a drone, or the like. The mobile object may include a body and a lighting device provided in the body. The lighting device may emit light to make the position of the body recognizable. The lighting device includes the organic light-emitting element according to the present embodiment.

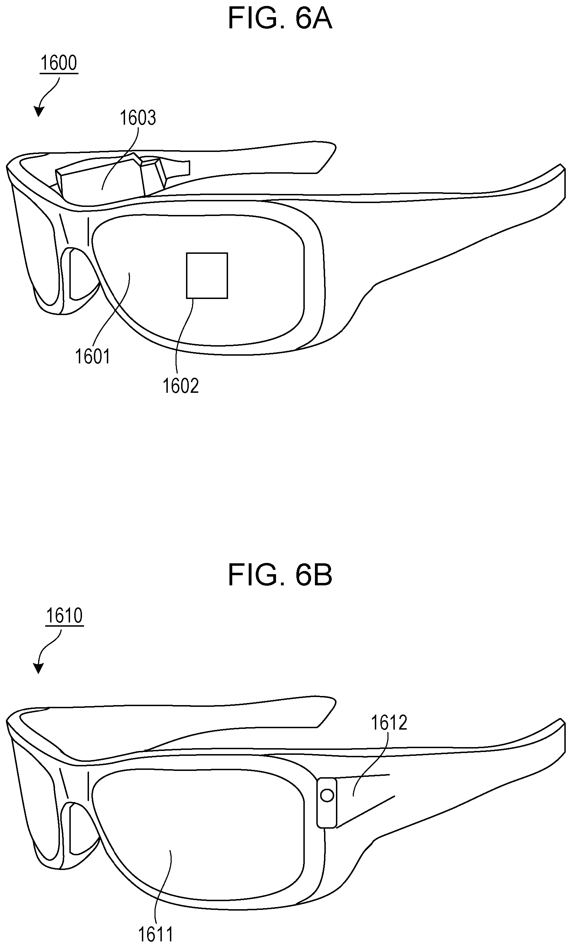

Referring to Fig. 6A and Fig. 6B, application examples of the above-described display devices of the embodiments will be described. The display devices can be applied to systems wearable as wearable devices such as smart glasses, HMDs, and smart contact lenses. Image pickup display devices used in such application examples include an image pickup device configured to perform photoelectric conversion of visible light and a display device configured to emit visible light.

Fig. 6A is a schematic view illustrating an example of a wearable device according to an embodiment of the present invention. Referring to Fig. 6A, glasses 1600 (smart glasses) according to one application example will be described. An image pickup device 1602 such as a CMOS sensor or a SPAD is provided on the front surface side of a lens 1601 of the glasses 1600. The display device of each of the above-described embodiments is provided on the back surface side of the lens 1601.

The glasses 1600 further include a controller 1603. The controller 1603 functions as a power source that supplies power to the image pickup device 1602 and the display device. The controller 1603 also controls operations of the image pickup device 1602 and the display device. An optical system for collecting light onto the image pickup device 1602 is formed in the lens 1601.

Fig. 6B is a schematic view illustrating another example of the wearable device according to an embodiment of the present invention. Referring to Fig. 6B, glasses 1610 (smart glasses) according to one application example will be described. The glasses 1610 include a controller 1612, and the controller 1612 includes an image pickup device corresponding to the image pickup device 1602 of Fig. 6A and a display device. An optical system for projecting light emission from the image pickup device and the display device in the controller 1612 is formed in a lens 1611, and an image is projected on the lens 1611. The controller 1612 functions as a power source that supplies power to the image pickup device and the display device, and also controls operations of the image pickup device and the display device.

The controller 1612 may include a gaze detection unit for detecting gaze of the wearer. The gaze detection may be performed using infrared radiation. An infrared light-emitting unit emits infrared light toward the eyeball of the user who is gazing at a displayed image. The image pickup unit including a light receiving element detects reflected light of the emitted infrared light from the eyeball to obtain a captured image of the eyeball. A reduction unit that reduces light from the infrared light-emitting unit to the display unit in plan view reduces degradation of image quality. The user's gaze toward the displayed image is detected from the captured image of the eyeball obtained by infrared image pickup. For the gaze detection using the captured image of the eyeball, any publicly known method can be applied. An example can be a gaze detection method based on a Purkinje image generated by reflection of irradiation light on the cornea. More specifically, gaze detection processing based on a pupil-corneal reflection method is performed. The pupil-corneal reflection method is used to calculate a gaze vector representing the orientation (rotation angle) of the eyeball based on an image of the pupil and a Purkinje image included in the captured image of the eyeball, to thereby detect the user's gaze.

The display device according to an embodiment of the present invention may include an image pickup device including a light receiving element and may control a displayed image of the display device based on the user's gaze information from the image pickup device. Specifically, the display device determines, based on gaze information, a first field-of-view region gazed at by the user and a second field-of-view region other than the first field-of-view region. The first field-of-view region and the second field-of-view region may be determined by the controller of the display device, or the display device may receive the regions determined by an external controller. In a display region of the display device, the display resolution of the first field-of-view region may be controlled to be higher than the display resolution of the second field-of-view region. In other words, the resolution of the second field-of-view region may be made lower than that of the first field-of-view region.

The display region includes a first display region and a second display region different from the first display region, and a region having a higher priority is determined, based on the gaze information, from among the first display region and the second display region. The first field-of-view region and the second field-of-view region may be determined by the controller of the display device, or the display device may receive the regions determined by an external controller. The resolution of the higher-priority region may be controlled to be higher than the resolution of the non-higher-priority region. In other words, the resolution of the region having a relatively lower priority may be reduced.

Note that AI may be used for determining the first field-of-view region or the high-priority region. The AI may be a model configured to estimate a gaze angle and the distance to the gaze target from an image of the eyeball, using, as training data, the image of the eyeball and the actual gaze direction of the eyeball in the image. The AI program may be included in the display device, the image pickup device, or an external device. When it is included in the external device, it is transmitted to the display device via communication.

When display control is performed based on visual recognition detection, application to smart glasses further including an image pickup device for image pickup of the outside is preferred. The smart glasses can display captured outside information in real time.

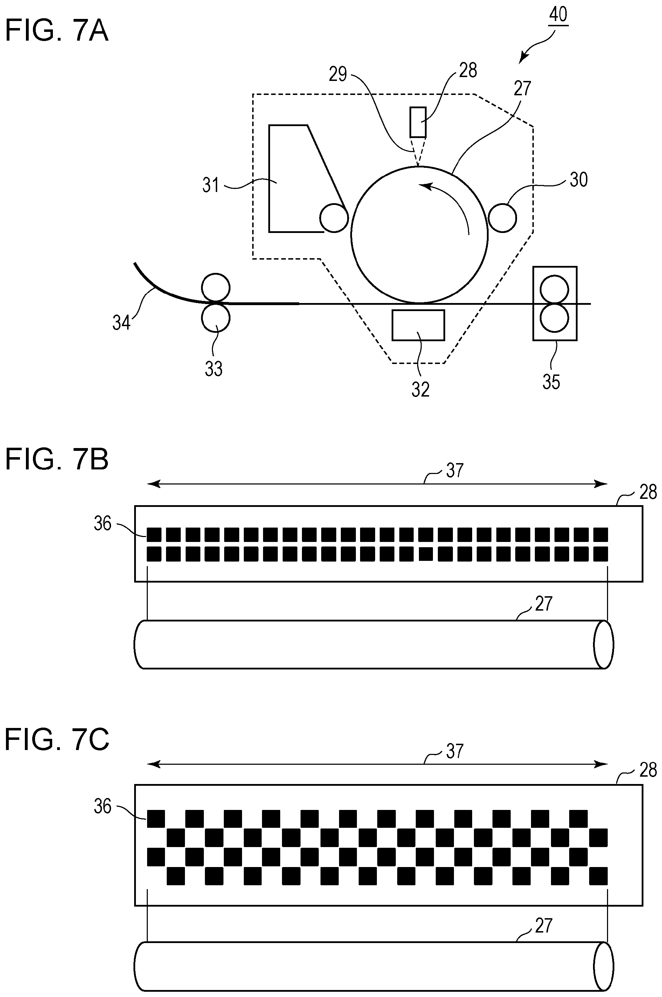

Fig. 7A is a schematic view illustrating an example of an image forming apparatus according to an embodiment of the present invention. An image forming apparatus 40 is an electrophotographic image forming apparatus including a photosensitive member 27, an exposure light source 28, a charging unit 30, a developing unit 31, a transfer unit 32, a conveying roller 33, and a fixing unit 35. The exposure light source 28 emits light 29 to form an electrostatic latent image on the surface of the photosensitive member 27. The exposure light source 28 includes the organic light-emitting element according to the present embodiment. The developing unit 31 includes toner or the like. The charging unit 30 charges the photosensitive member 27. The transfer unit 32 transfers the developed image onto a recording medium 34. The conveying roller 33 conveys the recording medium 34. The recording medium 34 may be paper, for example. The fixing unit 35 fixes the image formed on the recording medium 34.

Fig. 7B and Fig. 7C are schematic views illustrating the exposure light source 28, and illustrating configurations in which a plurality of light-emitting portions 36 are arranged on an elongated substrate. Arrows 37 indicate a direction parallel to the axis of the photosensitive member and a row direction in which the organic light-emitting elements are arranged. This row direction is the same as the direction of the axis of rotation of the photosensitive member 27. This direction can also be referred to as the longitudinal direction of the photosensitive member 27. Fig. 7B illustrates a configuration in which the light-emitting portions 36 are arranged in the longitudinal direction of the photosensitive member 27. Fig. 7C illustrates a configuration different from that of Fig. 7B, in which the light-emitting portions 36 are alternately arranged in the row direction in each of a first row and a second row. The first row and the second row are disposed at different positions in the column direction. In the first row, a plurality of light-emitting portions 36 are arranged at intervals. The second row includes light-emitting portions 36 at positions corresponding to the intervals between the light-emitting portions 36 in the first row. Thus, a plurality of light-emitting portions 36 are arranged at intervals also in the column direction. The arrangement in Fig. 7C can also be referred to as, for example, a grid arrangement, a staggered arrangement, or a checkerboard pattern.

As has been described, use of apparatuses or devices using the organic light-emitting element according to the present embodiment can achieve stable display with good image quality even for long-time display. In addition, use of apparatuses or devices using the organic light-emitting element according to the present embodiment can achieve both good visibility outdoors and power-saving display due to high-efficiency and high-luminance light output.

### EXAMPLES

Hereinafter, the present invention will be described by way of Examples. However, the present invention is not limited thereto.

### [Example 1 (Synthesis of Exemplified Compound B27)]

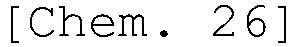

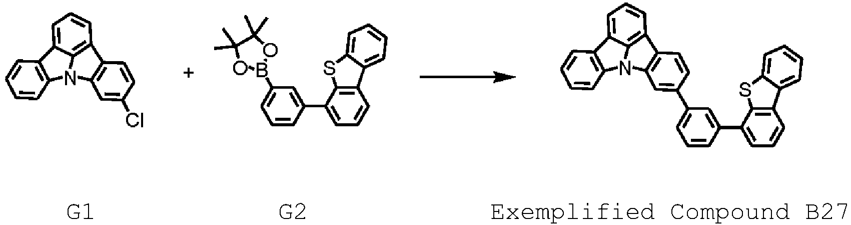

A 500 ml recovery flask was charged with the following reagents and solvents.
Compound G1: 1.00 g (3.63 mmol)
Compound G2: 1.40 g (3.63 mmol)
Pd(OAc)₂: 25 mg (0.11 mmol)
s-phos: 163 mg (0.36 mmol)
K₃PO₄: 1.54 g (7.26 mmol)
Toluene: 30 ml
Water: 1 ml

Next, the reaction solution was heated to 90°C under a nitrogen stream and stirred at this temperature (90°C) for 5 hours. After completion of the reaction, methanol was added and filtration was performed to obtain a crude product as a residue. This was purified by silica gel column chromatography (chlorobenzene) and recrystallized from xylene to obtain 907 mg of Exemplified Compound B27 (yield: 65%).

Exemplified Compound B27 was subjected to mass spectrometry using MALDI-TOF-MS (Autoflex LRF manufactured by Bruker Corporation).

### [MALDI-TOF-MS]

| | |
|---|---|
| Measured value: m/z = 500 | Calculated value: C₃₆H₂₁NS = 500 |

### [Examples 2 to 28 (Synthesis of Exemplified Compounds)]

Exemplified Compounds were synthesized as in Example 1 except that, in Example 1, the raw material G1 was changed to a raw material 1 and the raw material G2 was changed to a raw material 2. However, in Examples 22 to 24, the molar ratio of the raw material 1 to the raw material 2 was changed to raw material 1:raw material 2 = 2:1. The raw material 1 and the raw material 2 of each Example will be described in Table 5 to Table 8. In addition, measured values m/z of mass spectrometry results measured as in Example 1 will be described in Table 5 to Table 8.

**[Table 5]**

| Example | Exemplified Compound | Raw material 1 | Raw material 2 | m/z |
|---|---|---|---|---|
| 2 | A18 | 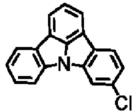 | 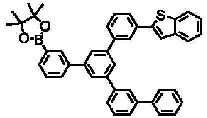 | 754 |
| 3 | B7 | 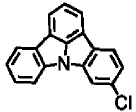 | 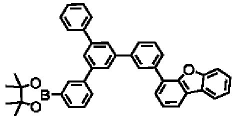 | 712 |
| 4 | B13 | 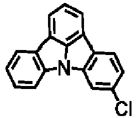 | 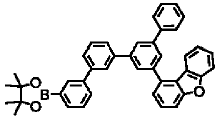 | 712 |
| 5 | B19 | 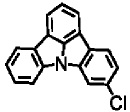 | 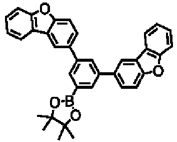 | 650 |
| 6 | B22 | 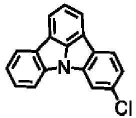 | 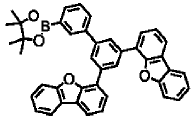 | 726 |
| 7 | B31 | 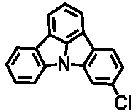 | 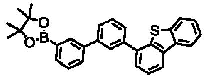 | 576 |
| 8 | B32 | 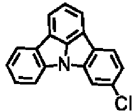 | 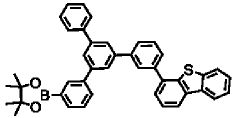 | 728 |
| 9 | B34 | 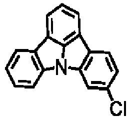 | 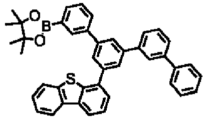 | 728 |

**[Table 6]**

| Example | Exemplified Compound | Raw material 1 | Raw material 2 | m/z |
|---|---|---|---|---|
| 10 | B41 | 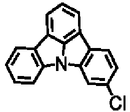 | 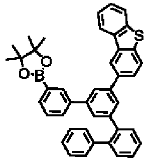 | 728 |
| 11 | B43 | 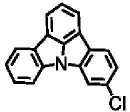 | 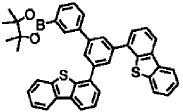 | 682 |
| 12 | B44 | 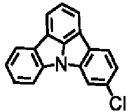 | 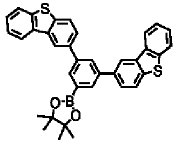 | 682 |
| 13 | B47 | 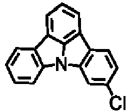 | 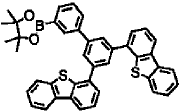 | 758 |
| 14 | B48 | 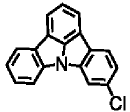 | 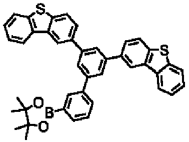 | 758 |
| 15 | B62 | 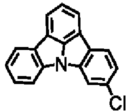 | 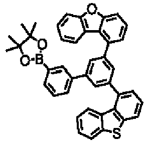 | 742 |
| 16 | B66 | 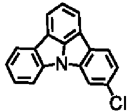 | 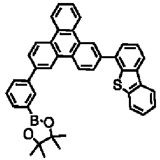 | 726 |

**[Table 7]**

| Example | Exemplified Compound | Raw material 1 | Raw material 2 | m/z |
|---|---|---|---|---|
| 17 | C6 | 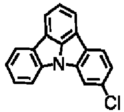 | 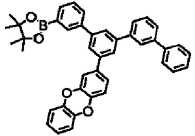 | 728 |
| 18 | C15 | 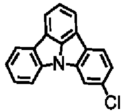 | 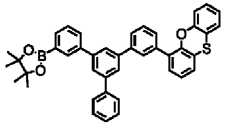 | 744 |
| 19 | C24 | 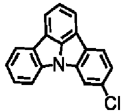 | 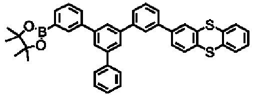 | 760 |
| 20 | C38 | 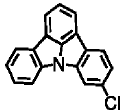 | 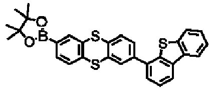 | 638 |
| 21 | C45 | 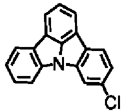 | 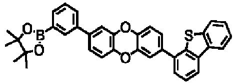 | 682 |

**[Table 8]**

| Example | Exemplified Compound | Raw material 1 | Raw material 2 | m/z |
|---|---|---|---|---|
| 22 | D3 | 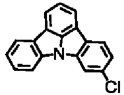 | 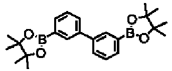 | 633 |
| 23 | D7 | 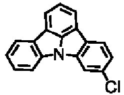 | 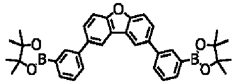 | 799 |
| 24 | D9 | 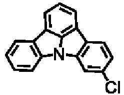 | 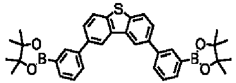 | 815 |
| 25 | E9 | 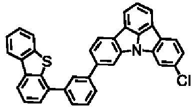 | 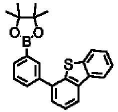 | 758 |
| 26 | E11 | 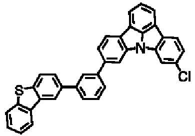 | 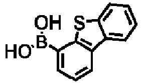 | 682 |
| 27 | E12 | 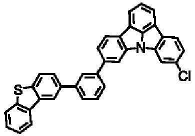 | 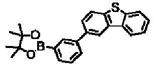 | 758 |
| 28 | E28 | 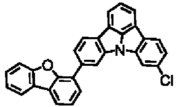 | 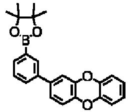 | 666 |

### [Example 29]

A bottom-emission type organic light-emitting element having a structure in which an anode, a hole injection layer, a hole transport layer, an electron blocking layer, a light-emitting layer, a hole blocking layer, an electron transport layer, an electron injection layer, and a cathode were sequentially formed on a substrate was produced.

First, an ITO film was formed on a glass substrate, and subjected to desired patterning processing to form an ITO electrode (anode). At this time, the ITO electrode was formed with a film thickness of 100 nm. The substrate on which the ITO electrode was formed in this manner was used as an ITO substrate in the following steps. Next, vacuum deposition by resistance heating was performed in a vacuum chamber at 1.33 × 10⁻⁴ Pa to continuously form organic compound layers and an electrode layer described in Table 9 on the ITO substrate. Note that, at this time, the opposing electrode (a metal electrode layer, a cathode) was formed so as to have an electrode area of 3 mm².

**[Table 9]**

| | Material | | | Film thickness (nm) |
|---|---|---|---|---|
| Cathode | Al | | | 100 |
| Electron injection layer (EIL) | LiF | | | 1 |
| Electron transport layer (ETL) | ET2 | | | 20 |
| Hole blocking layer (HBL) | ET11 | | | 20 |
| Light-emitting layer (EML) | Host | B7 | Mass ratio B7:GD11 = 90:10 | 20 |
| | Guest | GD11 | | |
| Electron blocking layer (EBL) | HT19 | | | 15 |
| Hole transport layer (HTL) | HT3 | | | 30 |
| Hole injection layer (HIL) | HT16 | | | 5 |

For the obtained element, characteristics of the element were measured and evaluated. The light-emitting element was found to have a maximum external quantum efficiency (E.Q.E.) of 13%. Furthermore, a continuous driving test was performed at a current density of 100 mA/cm², and the time to a 5% decrease in luminance was measured. When the time to a 5% decrease in luminance of Comparative Example 1 was taken as 1.0, the luminance decrease ratio of the present Example was 1.3.

In EXAMPLES, the measurement instruments employed were specifically as follows: the current-voltage characteristics were measured using a pA meter 4140B manufactured by Hewlett-Packard Company, and the light emission luminance was measured using a BM7 manufactured by TOPCON CORPORATION.

### [Examples 30 to 52 and Comparative Example 1]

The same procedures as in Example 29 were performed except for appropriate changes to compounds described in Table 10, to produce organic light-emitting elements. For the obtained elements, characteristics of the elements were measured and evaluated as in Example 29. The measurement results will be described in Table 10.

**[Table 10]**

| | HIL | HTL | EBL | EML | | HBL | ETL | E.Q.E [%] | Luminance decrease ratio |
|---|---|---|---|---|---|---|---|---|---|
| | | | | Host | Guest | | | | |
| Example 30 | HT16 | HT3 | HT19 | B13 | GD11 | ET11 | ET2 | 13 | 1.3 |
| Example 31 | HT16 | HT2 | HT15 | B19 | GD11 | ET12 | ET2 | 12 | 1.2 |
| Example 32 | HT16 | HT3 | HT19 | B22 | GD11 | ET11 | ET2 | 13 | 1.3 |
| Example 33 | HT16 | HT3 | HT19 | B27 | GD11 | ET11 | ET2 | 13 | 1.3 |
| Example 34 | HT16 | HT3 | HT19 | B31 | GD11 | ET11 | ET15 | 13 | 1.3 |
| Example 35 | HT16 | HT3 | HT19 | B32 | GD11 | ET11 | ET2 | 13 | 1.4 |
| Example 36 | HT16 | HT3 | HT19 | B34 | GD11 | ET11 | ET2 | 13 | 1.4 |
| Example 37 | HT16 | HT2 | HT15 | B41 | GD11 | ET12 | ET15 | 14 | 1.2 |
| Example 38 | HT16 | HT3 | HT19 | B43 | GD11 | ET11 | ET15 | 13 | 1.3 |
| Example 39 | HT16 | HT3 | HT19 | B44 | GD11 | ET11 | ET2 | 13 | 1.2 |
| Example 40 | HT16 | HT3 | HT19 | B47 | GD11 | ET11 | ET2 | 13 | 1.5 |
| Example 41 | HT16 | HT3 | HT19 | B48 | GD11 | ET11 | ET2 | 13 | 1.4 |
| Example 42 | HT16 | HT3 | HT19 | B66 | GD11 | ET11 | ET2 | 13 | 1.2 |
| Example 43 | HT16 | HT2 | HT15 | C6 | GD11 | ET12 | ET15 | 13 | 1.2 |
| Example 44 | HT16 | HT3 | HT19 | C24 | GD11 | ET11 | ET2 | 12 | 1.1 |
| Example 45 | HT16 | HT3 | HT19 | C38 | GD11 | ET11 | ET2 | 13 | 1.2 |
| Example 46 | HT16 | HT3 | HT19 | D3 | GD11 | ET11 | ET2 | 13 | 1.2 |
| Example 47 | HT16 | HT3 | HT19 | D7 | GD11 | ET11 | ET2 | 12 | 1.2 |
| Example 48 | HT16 | HT3 | HT19 | D9 | GD11 | ET11 | ET2 | 12 | 1.2 |
| Example 49 | HT16 | HT3 | HT19 | E9 | GD11 | ET11 | ET2 | 13 | 1.2 |
| Example 50 | HT16 | HT3 | HT19 | E11 | GD11 | ET11 | ET2 | 13 | 1.2 |
| Example 51 | HT16 | HT3 | HT19 | E12 | GD11 | ET11 | ET2 | 13 | 1.2 |
| Example 52 | HT16 | HT3 | HT19 | E28 | GD11 | ET11 | ET2 | 12 | 1.1 |
| Comparative Example 1 | HT16 | HT3 | HT19 | Comparative Compound 1-a | GD11 | ET11 | ET2 | 12 | 1.0 |

As described above, the Exemplified Compounds according to the present embodiment, which include an indolocarbazole unit and have heteroaryl units at two terminals of the molecule, provide improved compatibility with the guest molecule, shorten the intermolecular distance, and provide high energy transfer efficiency. Therefore, elements having high efficiency and improved durability characteristics can be provided.

The present invention is not limited to the above-described embodiments, and various changes and modifications can be made without departing from the spirit and scope of the present invention. Accordingly, the following claims are appended in order to make public the scope of the present invention.

This application claims priority based on Japanese Patent Application No. 2023-185492 filed October 30, 2023, which is hereby incorporated by reference herein in its entirety.

### Reference Signs List

- 1: interlayer insulating layer
- 2: first electrode
- 3: insulating layer
- 4: organic compound layer
- 5: second electrode
- 6: protective layer
- 7: color filter
- 10: sub-pixel
- 11: substrate
- 12: insulating layer
- 13: gate electrode
- 14: gate insulating film
- 15: semiconductor layer
- 16: drain electrode
- 17: source electrode
- 18: TFT
- 19: insulating film
- 20: contact hole
- 21: anode
- 22: organic compound layer
- 23: cathode
- 24: first protective layer
- 25: second protective layer
- 26: organic light-emitting element
- 100: display device

## Claims

1. An organic compound represented by a general formula [1] or [2] below: 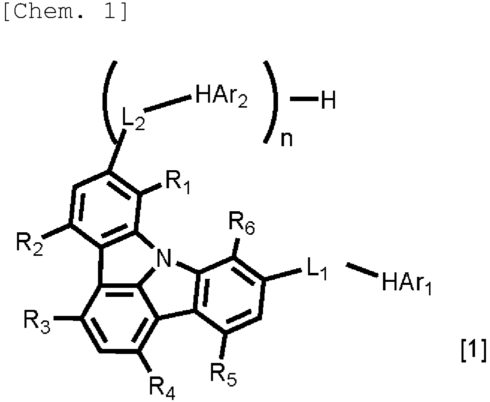
wherein, in the general formula [1], L₁ and L₂ are each independently a direct bond or a linking group composed of at least one selected from the group consisting of a substituted or unsubstituted benzene residue, a substituted or unsubstituted naphthalene residue, a substituted or unsubstituted phenanthrene residue, a substituted or unsubstituted triphenylene residue, and a substituted or unsubstituted residue of a compound represented by a general formula [a] below: 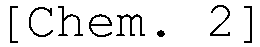 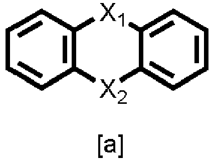
in the general formula [a], X₁ and X₂ are each independently selected from O, S, Se, and Te,
HAr₁ is selected from groups represented by general formulas [b] to [d] below, and HAr₂ is selected from groups represented by general formulas [e] to [g] below, groups represented by general formulas [b] to [g] may further have a fused ring, 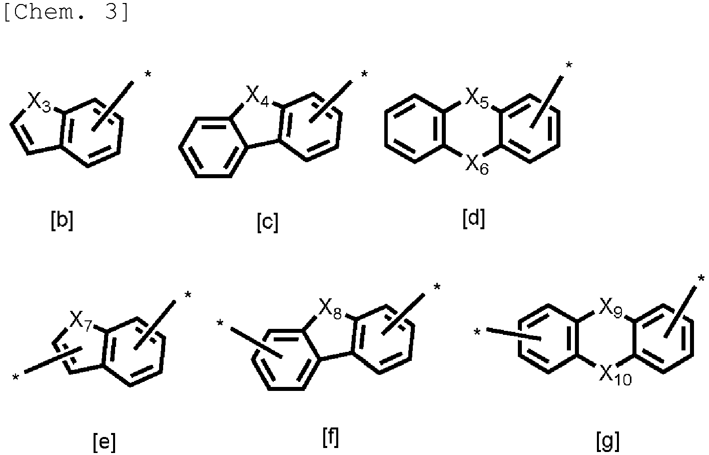
in the general formulas [b] to [g], X₃ to X₁₀ are each independently selected from O, S, Se, and Te; in the general formulas [b] to [d], * represent a bonding position with L₁; in the general formulas [e] to [g], * represent a bonding position with L₂ or H,
R₁ to R₆ are each independently selected from a hydrogen atom, a deuterium atom, a halogen atom, a substituted or unsubstituted alkyl group, a substituted or unsubstituted alkoxy group, and a substituted or unsubstituted silyl group,
n is 0 or 1, 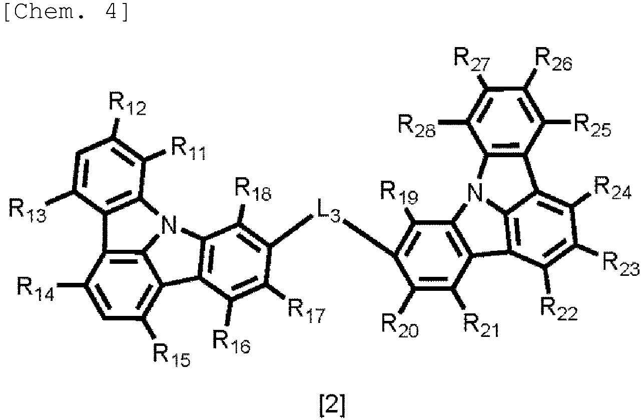
in the general formula [2], L₃ is a linking group composed of at least one selected from the group consisting of a substituted or unsubstituted benzene residue, a substituted or unsubstituted naphthalene residue, a substituted or unsubstituted phenanthrene residue, a substituted or unsubstituted triphenylene residue, and substituted or unsubstituted groups represented by general formulas [h] to [j] below: 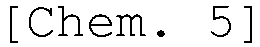 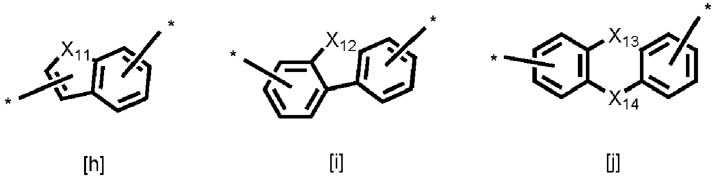
in the general formulas [h] to [j], X₁₁ to X₁₄ are each independently selected from O, S, Se, and Te, * represent a bonding position, and
R₁₁ to R₂₈ are each independently selected from a hydrogen atom, a deuterium atom, a halogen atom, a substituted or unsubstituted alkyl group, a substituted or unsubstituted alkoxy group, and a substituted or unsubstituted silyl group.

2. The organic compound according to claim 1, wherein, in the general formula [1], L₁ and L₂ are each independently a direct bond or a linking group composed of at least one selected from the group consisting of a substituted or unsubstituted benzene residue having a valence of three or less, a substituted or unsubstituted naphthalene residue having a valence of three or less, a substituted or unsubstituted phenanthrene residue having a valence of three or less, a substituted or unsubstituted triphenylene residue having a valence of three or less, and a substituted or unsubstituted residue having a valence of three or less of the compound represented by the general formula [a], and
in the general formula [2], L₃ is a linking group composed of at least one selected from the group consisting of a substituted or unsubstituted benzene residue having a valence of three or less, a substituted or unsubstituted naphthalene residue having a valence of three or less, a substituted or unsubstituted phenanthrene residue having a valence of three or less, a substituted or unsubstituted triphenylene residue having a valence of three or less, and the substituted or unsubstituted groups represented by the general formulas [h] to [j].

3. The organic compound according to claim 1 or 2, wherein, in the general formula [1], L₁ and L₂ are each independently a linking group composed of at least one selected from the group consisting of a substituted or unsubstituted benzene residue, a substituted or unsubstituted naphthalene residue, a substituted or unsubstituted phenanthrene residue, and a substituted or unsubstituted triphenylene residue.

4. The organic compound according to claim 1 or 2, wherein the benzene residue is a residue bonded at a meta position.

5. The organic compound according to claim 3, wherein, in the general formula [1], L₁ and L₂ are a linking group composed of one or more m-phenylene groups.

6. The organic compound according to claim 1 or 2, wherein, in the general formula [1], n is 0.

7. The organic compound according to claim 1 or 2, wherein, in the general formula [1], HAr₁ is a group represented by the general formula [c], and HAr₂ is a group represented by the general formula [f].

8. The organic compound according to claim 1 or 2, wherein, in the general formula [1], HAr₁ is a group represented by the general formula [d], and HAr₂ is a group represented by the general formula [g].

9. The organic compound according to claim 1 or 2, wherein, in the general formula [1], R₁ to R₆ are hydrogen atoms, and, in the general formula [2], R₁₁ to R₂₈ are hydrogen atoms.

10. An organic light-emitting element comprising a first electrode, a second electrode, and an organic compound layer disposed between the first electrode and the second electrode, wherein at least one layer of the organic compound layer contains the organic compound according to claim 1 or 2.

11. The organic light-emitting element according to claim 10, wherein the organic compound layer includes a light-emitting layer, and the light-emitting layer contains the organic compound.

12. The organic light-emitting element according to claim 11, wherein the light-emitting layer further contains a phosphorescent light-emitting compound.

13. The organic light-emitting element according to claim 12, wherein the light-emitting layer further contains an electron-transporting compound.

14. The organic light-emitting element according to claim 11, wherein another light-emitting layer disposed on the light-emitting layer is further included, and the other light-emitting layer is configured to emit light of a color different from an emission color of the light-emitting layer.

15. The organic light-emitting element according to claim 14, configured to emit white light.

16. A display device comprising a plurality of pixels,
wherein at least one of the plurality of pixels includes the organic light-emitting element according to claim 10 and a transistor connected to the organic light-emitting element.

17. A photoelectric conversion apparatus comprising an optical unit including a plurality of lenses, an image pickup element configured to receive light that has passed through the optical unit, and a display unit configured to display an image captured by the image pickup element,
wherein the display unit includes the organic light-emitting element according to claim 10.

18. An electronic apparatus comprising a display unit including the organic light-emitting element according to claim 10, a housing in which the display unit is disposed, and a communication unit that is disposed in the housing and configured to communicate with an external device.

19. A lighting apparatus comprising a light source including the organic light-emitting element according to claim 10, and a light diffusion unit or an optical filter configured to transmit light emitted from the light source.

20. A mobile object comprising a lighting device including the organic light-emitting element according to claim 10, and a body in which the lighting device is disposed.

21. An image forming apparatus comprising a photosensitive member and an exposure light source configured to expose the photosensitive member,
wherein the exposure light source includes the organic light-emitting element according to claim 10.
